# EUROPEAN PATENT APPLICATION

(11) **EP 1 920 774 A1**
(43) Date of publication of application: **14.05.2008**
(21) Application number: 06781936.7
(22) Date of filing: 28.07.2006
(51) Int. Cl.: A61K 31/702, A61K 8/00, A61K 45/00, A61P 37/02

(54) **COMPOSITION CONTAINING FUCOIDAN OR FUCOIDAN HYDROLYSATE AND IMMUNOPOTENTIATING MATERIAL**

(30) Priority: 29.07.2005 JP 2005222254
(71) Applicant: SUNTORY LIMITED, Osaka-shi, Osaka 530-8203 (JP); Tropical Technology Center Ltd., Gushikawa-shi, Okinawa 904-2234 (JP)
(72) Inventor: NONAKA, Yuji, Osaka 5670894 (JP); YASUMOTO, Takeshi, Miyagi 9810952 (JP); NAOKI, Hideo, Sakai-shi, Osaka 592-8345 (JP); KUSUMOTO, Toshihide, Kagoshima 8900045 (JP)
(74) Representative: Vossius & Partner
(86) International application number: PCT/JP2006/315026
(87) International publication number: WO 2007/013613

(57) **Abstract**

Materials are provided that potentiate an immuno-stimulating activity of materials such as lactic acid bacteria. Such materials contribute to reducing the use of immuno-stimulating materials. It also contributes to shortening the time taken to select lactic acid bacteria that have a strong immuno-stimulating activity and preferred fermentation characteristics. For the purposes, fucoidan or a product obtained by acid hydrolyzing fucoidan (or oligosaccharides derived from it) is used in combination with an immuno-stimulating material. This combination synergistically potentiates the immunomodulatory activities or immuno-stimulating activities of the ingredients.

## Description

### TECHNICAL FIELD

The present invention relates to compositions that comprise fucoidan or a fucoidan hydrolysate and an immuno-stimulating material, and which can be used in foods, beverages, pharmaceuticals, health foods, physiologically functional foods and cosmetics with a view to enhancing immunity, regulating immune functions, etc.

### BACKGROUND ART

### Immuno-stimulating materials

Recent years, in the field of health foods, materials having an immuno-stimulating action are drawing attention and a lot of health foods containing such materials are being developed. These materials are typically used for the specific purpose of recovery of immunity if this has dropped due to aging, stress, disease and other factors, and they include, for example, microorganisms such as lactic acid bacteria (lactic acid coccus and lactic acid bacillus), yeasts and fungi; mushrooms such as *shiitake* mushroom, hen-of-the-woods, Ganoderma lucidum, and Agaricus blazei; sea weeds; and Chinese herbal medications.

### Lactic acid bacteria

Among the immuno-stimulating materials mentioned above, lactic acid bacteria are drawing particular attention for their immuno-stimulating activity. For example, a few of them have been reported to have various actions such as enhancement of the NK (natural killer) activity and improvement of the Th1/Th2 balance. In addition, lactic acid bacteria are known to be highly safe as evidenced by many years of their use in foods and beverages. Therefore, one may well consider lactic acid bacteria to be a desirable means as an immunomodulator.

The immuno-stimulating action of lactic acid bacteria may be potentiated by increasing their content in foods and beverages. However, lactic acid bacteria added in large amounts to foods or beverages have had a tendency to form a precipitate. For this reason, the use of lactic acid bacteria in foods and beverages has been practically limited to solids such as pickled foods and to those which do not bother customers even if they form a precipitate (as exemplified by yoghurt and milk beverages). Even if more lactic acid bacteria are grown by fermentation as in yoghurt, their count is saturated at a certain level, so there has been a limit on the effort that can be made to increase the content of lactic acid bacteria in foods and beverages. Furthermore, the manufacture of fermented foods has required the need to consider not only their functions but also other factors including the degree of fermentation, the flavor after fermentation, and the count of lactic acid bacteria after fermentation. Therefore, in order to develop physiologically functional foods or beverages using lactic acid bacteria, in particular, functional fermented foods or beverages that are intended to regulate immune function, it is essential to use those species of lactic acid bacteria that are superior not only in fermentation characteristics but also in the immunomodulatory activity. However, the fermentation characteristics of lactic acid bacteria are not at all correlated to their immunomodulatory activity. Hence, searching for the desired lactic acid bacteria is by no means easy to perform.

Several methods have recently been reported to be capable of enhancing the activity of immuno-stimulating materials such as lactic acid bacteria. Among them are the method of potentiating the immuno-stimulating action by performing mixed culture of 3 to 8 species of lactic acid bacteria and/or yeasts (Patent Document 1) and the method of potentiating the immuno-stimulating action by using lactic acid bacteria and a mushroom together (Patent Document 2).

### Fucoidan and fucoidan hydrolysates

Fucoidan is a sulfated polysaccharide contained in algae and it has been reported to have diverse activity as exemplified by anticoagulant action, lipid-rich blood clarifying action (i.e., the ability to remove cholesterol and lipid peroxide from blood), anti-tumor action, cancer metastasis suppressing action, and anti-AIDS virus infection. Among these actions, the ability to normalize the immune function of the living body is drawing particular attention and fucoidan is considered to be useful as a material for pharmaceuticals and health foods having the immunomodulatory activity. However, being a sulfated polysaccharide having an extremely high molecular weight, fucoidan presents some problems with absorbability, antigenicity, uniformity, anticoagulant activity, etc. when it is directly used in foods or beverages, pharmaceuticals, etc.

With a view to solving those problems, several attempts have been made to prepare low-molecular weight compounds from fucoidan. For example, the production of fucose-containing oligosaccharides by chemical synthesis has been reported (Non-patent Documents 1, 2 and 3), but they have not been preferred for use in foods, etc. since they are prepared by organic synthesis reaction.

Also reported are methods of hydrolyzing fucoidan to lower its molecular weight. For example, Patent Document 3 discloses a method of hydrolyzing fucoidan with an acid and it states that the obtained low-molecular weight fucoidan had a molecular weight distribution not greater than 5 x 10³. Patent Document 4 describes a method in which fucoidan is hydrolyzed without external addition of an acid to produce oligosaccharides. According to Patent Document 5, fucoidan is hydrolyzed with an enzyme.
Patent Document 1: JP 2005-68092 A;
Patent Document 2: JP 2004-51504 A;
Patent Document 3: JP H7-215990 A;
Patent Document 4: JP 2002-226496 A;
Patent Document 5: JP 2000-236889 A;
Non-patent Document 1: Carbohydrate research 4, 189-195 (1967);
Non-patent Document 2: Carbohydrate research 37, 75-79 (1974);
Non-patent Document 3: Carbohydrate research 41, 308-312 (1975).

### DISCLOSURE OF THE INVENTION

### PROBLEMS TO BE SOLVED BY THE INVENTION

The methods disclosed in Patent Documents 1 and 2 are simply characterized by combining two insoluble materials and they are not yet capable of preventing the formation of a precipitate. No study has ever been to develop materials that are effective in enhancing the activity of immuno-stimulating materials other than lactic acid bacteria.

If there were water-soluble materials capable of synergistically enhancing the activities of immuno-stimulating materials and lactic acid bacteria, not only would it be possible to develop immunomodulatory agents and foods or beverages having more potent beneficiary effects but also to reduce the use of immuno-stimulating materials and lactic acid bacteria without sacrificing their activities; as a result, one would be able to expand the range over which to develop foods containing immuno-stimulating materials and lactic acid bacteria. This will also contribute to reducing the burden of searching for lactic acid bacteria that have both good fermentation characteristics and the immunomodulatory activity. Considering the use of a substance in foods or beverages and in pharmaceuticals, it is required that the substance be highly safe and allow of simple manufacture.

An object, therefore, of the present invention is to provide a substance that may be combined with an immuno-stimulating material in such a way that their respective immunomodulatory activities can be potentiated.

The immunomodulatory activity as used herein does not mean the indirect regulation of immune function through improvement of an enterobacterium biota as achieved by xylooligosaccharides, but means the direct activation of immunocompetent cells as exhibited by several kinds of fucoidan.

Another object of the present invention is to provide a composition, an effective amount of which can be added in a precise manner to foods/beverages or pharmaceuticals.

### MEANS FOR SOLVING THE PROBLEMS

The present inventors found that, by combining fucoidan or an acid hydrolysate of fucoidan, preferably oligosaccharides, with desired lactic acid bacteria or immuno-stimulating material, their respective immunomodulatory or immuno-stimulating activities could be synergistically potentiated, and this has led to the accomplishment of the present invention. Hereinafter, the oligosaccharides derived from fucoidan are sometimes referred to as fucoidan oligosaccharides.

Therefore, the present invention relates to the following:
(1) A composition comprising fucoidan or a fucoidan hydrolysate and an immuno-stimulating material;
(2) The composition as recited in (1), wherein the hydrolysate is obtained by hydrolyzing fucoidan with 0.1 - 6.0 N acid at 25 - 100°C for 0.25 - 2.5 hours;
(3) The composition as recited in (2), wherein the hydrolysate is obtained by hydrolyzing fucoidan with 0.5 - 4.0 N acid at 30 - 90°C for 0.5 - 2.0 hours;
(4) The composition as recited in any one of (1) - (3), wherein the hydrolysis is effected with hydrochloric acid or sulfuric acid;
(5) The composition as recited in any one of (1) - (4), wherein the hydrolysate is an oligosaccharide;
(6) The composition as recited in (5), wherein the oligosaccharide is a di-, tri-, tetra- or penta-saccharide that are composed of at least one member of the group consisting of fucose, sulfated fucose, acetylated fucose and glucuronic acid;
(7) The composition as recited in (6), which contains as the oligosaccharide at least one member of the group consisting of the compounds represented by the following structural formulae (I), (II), (III), (IV), (V), (VI), (VII), (VIII), (IX), (X), and (XI):

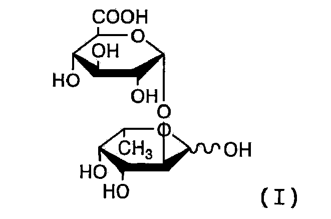

(molecular weight: 340);

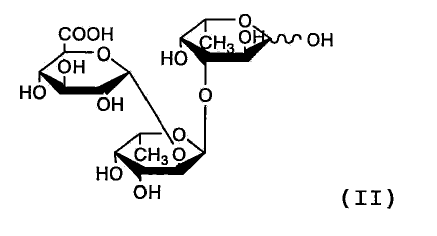

(molecular weight: 486);

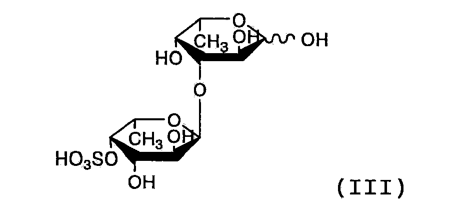

(molecular weight: 390);

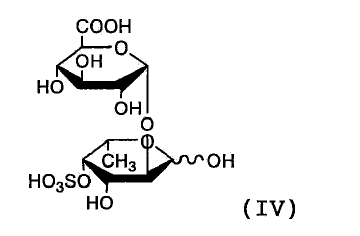

(molecular weight: 420);

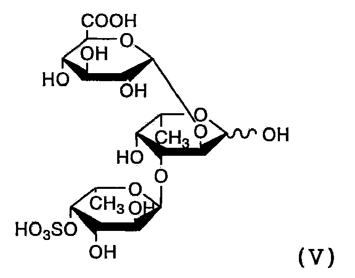

(molecular weight: 566);

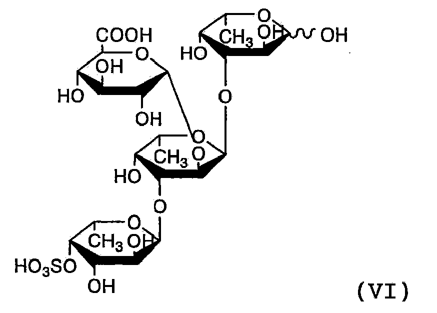

(molecular weight: 712);

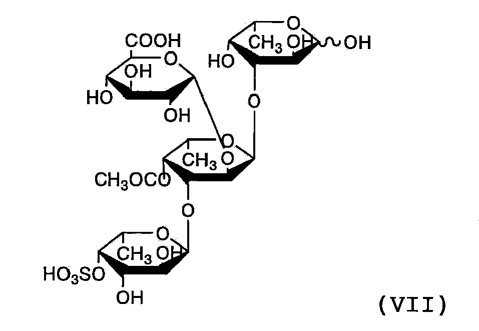

(molecular weight: 754);

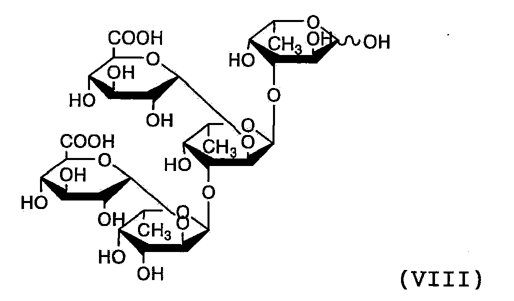

(molecular weight: 808);

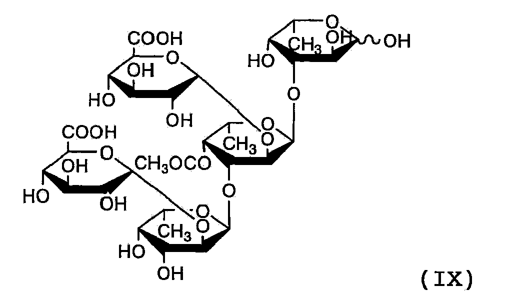

(molecular weight: 850);

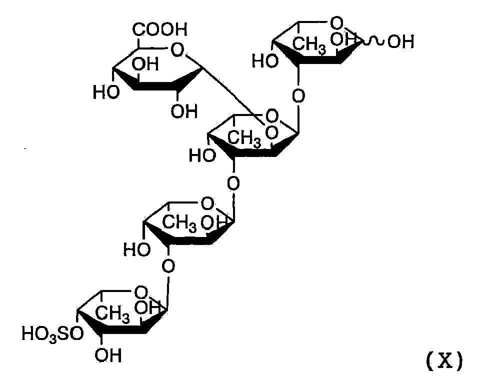

(molecular weight: 858);

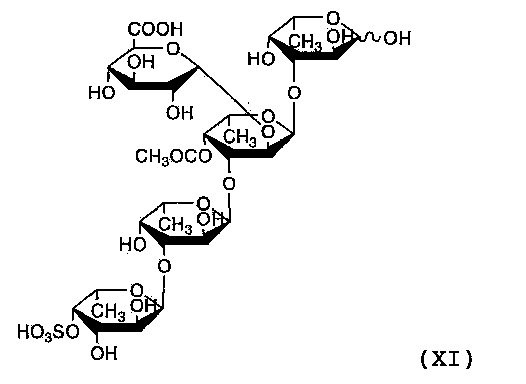

(molecular weight: 900);
(8) The composition as recited in any one of (1) - (7), wherein the immuno-stimulating material is at least one member of the group consisting of microorganisms such as lactic acid bacteria (lactic acid coccus and lactic acid bacillus), yeasts and fungi; mushrooms such as *shiitake* mushroom, hen-of-the-woods, Ganoderma lucidum, and Agaricus blazei; nucleic acid derived substances such as CpG motif containing immunogenic oligodeoxynucleotide (CpG-ODN) and PolyI:C; lipopolysaccharides (LPS) and β-glucan; sea weeds; Chinese herbal medications; cancer vaccines (e.g. MM46-tumor antigen); concanavalin A (ConA); and BRMs (biological response modifiers) such as Krestin (registered trademark), OK-432 (Picibanil, registered trademark), and Lentinan (registered trademark);
(9) The composition as recited in (8), wherein the immuno-stimulating material is a lactic acid bacterium;
(10) An immunomodulatory agent comprising the composition as recited in any one of (1) - (9);
(11) A food or a drink incorporating the composition as recited in any one of (1) - (9), added thereto;
(12) A food or a drink that comprise the composition as recited in any one of (1) - (9) and which has an indication stating that the good or dring has an immunomodulatory activity;
(13) A pharmaceutical composition comprising the composition as recited in any one of (1) - (9); and
(14) A cosmetic comprising the composition as recited in any one of (1) - (9).

### EFFECTS OF THE INVENTION

Fucoidan or a hydrolysate thereof and the immuno-stimulating material that are combined in accordance with the present invention offer the advantage of exhibiting its synergistically enhanced immunomodulatory activity or immuno-stimulating action as compared with the case where they are applied individually. This makes it possible to develop foods or beverages and pharmaceuticals that are more effective than those products which incorporate the conventional immuno-stimulating materials. Such high efficacy contributes to reducing the amount in which the immuno-stimulating material is to be added to foods and beverages or pharmaceuticals or cosmetics.

These advantages bear particular importance when lactic acid bacteria are used as an immuno-stimulating material. For instance, lactic acid bacteria of low water solubility may be prevented from precipitating if the amount of their addition is reduced. In addition, the step of selecting lactic acid bacteria that have both the immuno-stimulating activity and desired fermentation characteristics can be skipped. As a consequence, researchers engaged in the development of lactic acid bacteria containing products find themselves proceeding with their work in a more advantageous way than others.

Fucoidan or its hydrolysate which is a constituent of the composition of the present invention has been isolated from a natural foodstuff, so it has only a mild action and features an extremely high degree of safety.

Therefore, the composition of the present invention is very useful and the scope of its application covers not only foods and beverages but also pharmaceuticals and cosmetics.

If the composition contains as the oligosaccharide at least one member of the group consisting of the compounds represented by the structural formulae (I), (II), (III), (IV), (V), (VI), (VII), (VIII), (IX), (X), and (XI) shown above, its quality can be controlled in a simple, yet reliable manner. What is more, those fucoidan derived oligosaccharides have low molecular weights, so they are easy to handle and can be manufactured in a simple manner; in addition, because of their high safety, such oligosaccharides may be combined with immune-stimulating materials to prepare compositions that have a variety of uses including foods and beverages, as well as pharmaceuticals.

### BRIEF DESCRIPTION OF THE DRAWINGS

Fig. 1 is an HPLC chart showing a sugar compositional analysis of fucoidan as obtained from Okinawa Nemacystus decipiens by extraction with hot water;
Fig. 2A is a graph showing how the acid concentration affects the immuno-stimulating activity of fucoidan being hydrolyzed;
Fig. 2B is a graph showing how the acid concentration affects the immuno-stimulating activity of fucoidan being hydrolyzed;
Fig. 3A is a graph showing how the temperature affects the immuno-stimulating activity of fucoidan being hydrolyzed;
Fig. 3B is a graph showing how the temperature affects the immuno-stimulating activity of fucoidan being hydrolyzed;
Fig. 4 is a graph showing how the time affects the immuno-stimulating activity of fucoidan being hydrolyzed;
Fig. 5 shows a MS spectrum of the fucoidan oligosaccharide with a molecular weight of 340 that is represented by formula (I);
Fig. 6 shows a MS spectrum of the fucoidan oligosaccharide with a molecular weight of 486 that is represented by formula (II);
Fig. 7 shows a ¹H-NMR spectrum of the fucoidan oligosaccharide with a molecular weight of 340 that is represented by formula (I);
Fig. 8 shows a ¹³C-NMR spectrum of the fucoidan oligosaccharide with a molecular weight of 340 that is represented by formula (I);
Fig. 9 shows a ¹H-NMR spectrum of the fucoidan oligosaccharide with a molecular weight of 486 that is represented by formula (II);
Fig. 10 shows a ¹³C-NMR spectrum of the fucoidan oligosaccharide with a molecular weight of 486 that is represented by formula (II);
Fig. 11 shows a ¹H-NMR spectrum of the fucoidan oligosaccharide with a molecular weight of 390 that is represented by formula (III);
Fig. 12 shows a ¹³C-NMR spectrum of the fucoidan oligosaccharide with a molecular weight of 390 that is represented by formula (III);
Fig. 13 shows a ¹H-NMR spectrum of the fucoidan oligosaccharide with a molecular weight of 566 that is represented by formula (V);
Fig. 14 shows a ¹³C-NMR spectrum of the fucoidan oligosaccharide with a molecular weight of 566 that is represented by formula (V);
Fig. 15 shows a ¹H-NMR spectrum of the fucoidan oligosaccharide with a molecular weight of 712 that is represented by formula (VI);
Fig. 16 shows a ¹³C-NMR spectrum of the fucoidan oligosaccharide with a molecular weight of 712 that is represented by formula (VI);
Fig. 17 shows a ¹H-NMR spectrum of the fucoidan oligosaccharide with a molecular weight of 754 that is represented by formula (VII);
Fig. 18 shows a ¹³C -NMR spectrum of the fucoidan oligosaccharide with a molecular weight of 754 that is represented by formula (VII);
Fig. 19 shows a ¹H-NMR spectrum of the fucoidan oligosaccharide with a molecular weight of 808 that is represented by formula (VIII);
Fig. 20 shows a ¹³C-NMR spectrum of the fucoidan oligosaccharide with a molecular weight of 808 that is represented by formula (VIII);
Fig. 21 shows a ¹H-NMR spectrum of the fucoidan oligosaccharide with a molecular weight of 850 that is represented by formula (IX);
Fig. 22 shows a ¹³C-NMR spectrum of the fucoidan oligosaccharide with a molecular weight of 850 that is represented by formula (IX);
Fig. 23 shows a ¹H-NMR spectrum of the fucoidan oligosaccharide regenerated, which has a molecular weight of 754, and is represented by formula (VII);
Fig. 24 shows a TOF-MS spectrum of the fucoidan oligosaccharide regenerated, which has a molecular weight of 754, and is represented by formula (VII);
Fig. 25 shows an ESI-MS/MS spectrum of the fucoidan oligosaccharide regenerated, which has a molecular weight of 754, and is represented by formula (VII);
Fig. 26 shows a FAB-MS spectrum of the fucoidan oligosaccharide with a molecular weight of 420 that is represented by formula (IV);
Fig. 27 shows an MS/MS spectrum of the fucoidan oligosaccharide with a molecular weight of 420 that is represented by formula (IV);
Fig. 28 shows FAB-MS spectra of the fucoidan oligosaccharides with molecular weights of 858 and 900 that are represented by formulae (X) and (XI), respectively;
Fig. 29 shows a FAB MS/MS spectrum of the fucoidan oligosaccharide with a molecular weight of 858 that is represented by formula (X);
Fig. 30 shows a FAB MS/MS spectrum of the fucoidan oligosaccharide with a molecular weight of 900 that is represented by formula (XI);
Fig. 31 shows an ESI-MS chart obtained by hydrolyzing Okinawa Nemacystus decipiens and then subjecting the hydrolysate to fluorescence labeling with ABEE;
Fig. 32A is a graph showing how the combined use of a fucoidan oligosaccharide represented by formula (I) and lactic acid bacteria synergistically enhance the IFN-γ inducible activity on splenocytes;
Fig. 32B is a graph showing how the combined use of a fucoidan oligosaccharide represented by formula (II) and lactic acid bacteria synergistically enhance the IFN-γ inducible activity on splenocytes;
Fig. 33 is a graph showing how the combined use of fucoidan oligosaccharide mixtures and lactic acid bactera synergistically enhance the IFN-γ inducible activity on splenocytes;
Fig. 34 is a graph showing how the combined use of a fucoidan oligosaccharide and lactic acid bacteria synergistically enhance the IL-12 inducible activity on dendritic cells;
Fig. 35 is a graph showing how the combined use of a fucoidan oligosaccharide and lactic acid bacteria synergistically enhance the IL-10 inducible activity on dendritic cells;
Fig. 36 is a graph showing how the combined use of a fucoidan oligosaccharide and lactic acid bacteria synergistically enhance the CTL activity;
Fig. 37 is a graph showing the synergistic potentiation of IL-12 production as achieved by almost inactive lactic acid bacteria and a fucoidan hydrolysate;
Fig. 38 is a graph showing the synergistic potentiation of IFN production as achieved by an immuno-stimulating agent other than lactic acid bacteria and a fucoidan hydrolysate;
Fig. 39 is a graph demonstrating in vivo the synergistic potentiation of the NK activity as achieved by lactic acid bacteria and a fucoidan hydrolysate; and
Fig. 40 is a graph showing how the combined use of fucoidan and lactic acid bacteria synergistically enhance the IFN-γ inducible activity on splenocytes.

### BEST MODE FOR CARRYING OUT THE INVENTION

### Immuno-stimulating material

The immuno-stimulating material means a material capable of potentiating the immune response in animals and it may also be called an immunomodulating material. The immuno-stimulating materials that can be used in the present invention are not particularly limited and include the following known immuno-stimulating agents: microorganisms such as lactic acid bacteria (lactic acid coccus and lactic acid bacillus), yeasts and fungi; mushrooms such as *shiitake* mushroom, hen-of-the-woods, Ganoderma lucidum, and Agaricus blazei; nucleic acid derived substances such as CpG motif containing immunogenic oligodeoxynucleotide (CpG-ODN) and PolyI:C; lipopolysaccharides (LPS); sea weeds; Chinese herbal medications; cancer vaccines such as MM46-tumor antigen; concanavalin A (ConA); and BRMs (biological response modifiers) such as Krestin (registered trademark), OK-432 (Picibanil, registered trademark), and Lentinan (registered trademark).

CpG-ODN is a (short) oligodeoxynucleotide (ODN) containing a non-methylated cytosine-guanine dinucleotide (CpG) and CpG-ODN containing such CpG motif can directly activate antigen presenting cells. As a result, activation of NK cells and the like by CpG-ODN promotes the induction of Th1 type immunoresponse and generation of cytotoxic T lymphocytes (JP 2004-519453 A).

PolyI:C refers to a polynucleotide copolymer composed of inosinic acid and cytidylic acid and it may also be called polyinosinic-polycytidylic acid. It is generally known that in primates, PolyI:C functions to increase the concentration of interferons in body fluids.

Lypopolysaccharides are known as substances that can trigger the innate immunity.

MM46 is a kind of mammary cancer or tumor. Cancer vaccines such as antigens against MM46-tumor augment the immune function of attacking cancer cells, so they are used in cancer prevention or treatment.

ConA is a lectin derived from the seed of Jack bean. It is known to have a variety of immuno-stimulating actions.

BRM refers to drugs that are used in cancer therapy to potentiate or regulate immune functions and the like in the host, thereby modifying the interrelation between the tumor and the host to extract a therapeutic effect. Such drugs include OK-432, Krestin (registered trademark), and Lentinan (registered trademark). OK-432 is an anti-malignant tumor agent or a therapeutic for lymphangioma that contain as an active ingredient a freeze-dried powder or dried cells of penicillin-treated strain Su of Streptococcus pyogenes (group A, type 3). Krestin is a drug used in cancer treatment that contains as an active ingredient a group of protein-bound polysaccharides as obtained from the mycelium of Trametes versicolor. Lentinan is β-1,3-glucan obtained from *shiitake* mushroom.

### Lactic acid bacteria

Lactic acid bacteria that can be used in the present invention are not limited to any particular strains but they preferably belong to the genus Lactobacillus, among which plant-origin lactic acid bacteria that can ferment plant materials are particularly preferred. More preferred are Lactobacillus plantarum and Lactobacillus pentosus. If lactic acid bacteria are to be used in combination with fucoidan or its hydrolysate in the present invention, they may be viable or dead.

### Fucoidan

Fucoidan which is used as a starting material for the composition of the present invention may be of any structure and can be acquired from any kind of algae. Exemplary algae include sea weeds of the class Phaeophyceae comprising various orders such as Sphacelariales, Chordariales, Scytosiphonales, Dictyosiphonales, Cutleriales, Sporochnales, Dictyotales, Laminariales, and Fucales. Preferably, fucoidan derived from Nemacystus decipiens may be used and fucoidan derived from Okinawa Nemacystus decipiens is more preferred.

### Method of extracting fucoidan

Various methods for extracting fucoidan from algae have been studied and are now known extensively (including, for example, the method of using water as described in JP 10-245334 A, the method of using an acid as described in JP 10-195106 A, and the method of using an alkaline aqueous solvent as described in JP 2002-262788 A). Fucoidan to be used as a starting material in the present invention can be acquired by those known methods. In the present invention, fucoidan acquired by the following method may be employed.

Add 5-10 volumes of distilled water to Nemacystus decipiens (e.g. Okinawa Nemacystus decipiens) and perform extraction at 50-100°C for a few minutes to 5 hours, preferably at 60-90°C for 0.5-2.0 hours, more preferably at 80-90°C for about one hour. Cool the thus obtained algal extract, filter it by suction, and subject it to desalting and drying, whereby readily water-soluble fucoidan fractions are obtained. The thus obtained fucoidan fractions may be used in the next step with or without further purification.

Fucoidan to be used in the present invention is preferably an algal extract that is obtained in the manner described above; if desired, it may be used in a form as is naturally contained in algae. Fucoidan or an alga that contains it is then subjected to the subsequent hydrolyzing step to give the fucoidan hydrolysate.

### Fucoidan hydrolysate

The fucoidan hydrolysate refers to sugar derivatives embracing mono- and polysaccharides that are obtainable by hydrolyzing the sulfated polysaccharide fucoidan; it also refers to mixtures of such sugar derivatives. The fucoidan hydrolysate is preferably polysaccharides, more preferably oligosaccharides composed of 2-10 monosaccharides, and most preferably oligosaccharides composed of 2-5 monosaccharides. In the present invention, the oligosaccharides as mentioned above which can be obtained by hydrolyzing fucoindan are hereinafter sometimes referred to as fucoidan oligosaccharides. Monosaccharides that compose the oligosaccharides are preferably fucose, sulfated fucose, acetylated fucose and/or glucuronic acid. The compounds of the above formulae (I) - (XI) are particularly preferred oligosaccharides.

The fucoidan hydrolysate may be produced in the following manner.

### Production of the fucoidan hydrolysate

### (1) Hydrolysis of fucoidan

The fucoidan hydrolysate which is a constituent of the present invention is obtained by hydrolyzing fucoidan with an acid or an enzyme as described in Patent Documents 3-5. In the case of acid hydrolysis, either inorganic acids or organic acids may be used; exemplary inorganic acids are hydrochloric acid, sulfuric acid, phosphoric acid, and nitric acid; exemplary organic acids are acetic acid, citric acid, oxalic acid, succinic acid, formic acid, and propionic acid; preferred acids are hydrochloric acid and sulfuric acid. Preferably, the following conditions may be employed in acid hydrolysis.

The fucoidan-containing fraction thus obtained from algae in the manner described above or fucoidan itself is hydrolyzed with an acid, preferably hydrochloric acid, in an aqueous solvent containing 0.1-6.0 N, preferably 0.5-4.0 N, more preferably 0.5-2.0 N HCl, at 25-100°C, preferably 30-90°C, more preferably 50-80°C, for 0.1-3 hours, preferably 0.25-2.5 hours, more preferably 0.5-2.0 hours. The reaction product obtained is neutralized with a base, say, about 1 N NaOH, then desalted by a suitable means such as electrodialysis or gel filtration, and then dried (say, freeze-dried) to give a hydrolysate containing a mixture of fucoidan oligosaccharides.

The thus obtained hydrolysate may be directly used as a fucoidan hydrolysate but if desired, it may be further purified to have its activity enhanced.

### (2) Purification of the hydrolysate and isolation of oligosaccharides

The fucoidan hydrolysate can be purified by chromatography, recrystallization, dialysis, alcoholic precipitation and other methods that are used either independently or in combination, as typically described below.

First, the fucoidan hydrolysate containing the mixture of oligosaccharides is loaded on a chromatographic column filled with an anion-exchange resin, whereby fractions (neutral or acidic sugar fractions) that contain oligosaccharides free from sulfuric acid groups which simply pass through the column without being adsorbed are separated from fractions (sulfuated sugar fractions) that contain oligosaccharides rich in sulfuric acid groups which are eluted with an acidic eluting solution.

In the present invention, each of the fractions thus obtained is advantageously used as the fucoidan hydrolysate; but if desired, the fractions may be further purified so that the individual oligosaccharides are isolated before use.

The neutral or acidic sugar fractions may be subjected to gel filtration, thereby yielding a disaccharide represented by formula (I) and a trisaccharide represented by formula (II).

In contrast, the sulfated sugar fractions may be subjected to chromatography, thereby yielding the sulfated oligosaccharide (III), (IV), (V), (VI), (VII), (VIII), (IX), (X) or (XI) in an isolated form.

Each of the oligosaccharides may suitably be labeled or derivatized in order to further facilitate the steps of purification and structural analysis. For example, oligosaccharides may be labeled fluorescently by means of a reagent such as 4-aminobenzoic acid ethyl ester (ABEE) and this contributes to easy detection of the oligosaccharides. After separating each of the labeled oligosaccharides, the labeled portion may be removed, whereupon one can acquire a pure oligosaccharide.

### Combination of fucoidan or its hydrolysate and the immuno-stimulating material

In one aspect of the present invention, there is provided a composition comprising the thus obtained fucoidan or a hydrolysate thereof in combination with an immuno-stimulating material. Thus combined, fucoidan or the fucoidan hydrolysate and an immuno-stimulating material are potentiated in their respective immunomodulatory activity and/or immuno-stimulating action.

In this composition, a single fucoidan oligosaccharide may be used independently as the fucoidan hydrolysate in combination with an immuno-stimulating material (e.g. lactic acid bacteria). Alternatively, a plurality of fucoidan oligosaccharides, say three or more, may be combined with lactic acid bacteria.

If lactic acid bacteria are to be used in the combination of the present invention, they may be viable or dead. Oligosaccharides such as xylooligosaccharides which are known as prebiotics will potentiate the immunomodulatory activity primarily by promoting the proliferation of lactic acid bacteria. Compared to the oligosaccharides which are conventionally used as prebiotics, the hydrolysate of the present invention is advantageous in that they can also potentiate the immunomodulatory function of dead lactic acid bacteria.

Fucoidan or the fucoidan hydrolysate and the immuno-stimulating material are preferably used in a weight ratio from 1:1 to 10,000:1, more preferably from 250:1 to 1,000:1.

In another aspect, the present invention provides a method of using fucoidan or its hydrolysate and the immuno-stimulating material such that their immunomodulatory and/or immuno-stimulating actions are enhanced synergistically.

The combination according to the present invention may be applied in foods or beverages, pharmaceuticals, cosmetics and the like so that they are provided with an immunomodulatory activity.

### Immunomodulator

The immunomodulatory activity as referred to in the specification means the action of enhancing the immune response that has lowered in the living body and/or the action of suppressing the immune response that has increased in the living body. Therefore, the immunomodulatory activity covers both the immuno-stimulating action and the immunosuppressing action.

The immunomodulatory activity as referred to in the specification can be measured by methods known in the art. For example, it can be measured as the activity of inducing cytokines. Cytokines used for this purpose include interferon-γ, interleukin 10, interleukin 12, and the like. Other indices that may be used to measure the immunomodulatory activity include the maturation of dendritic cells which are involved in immune response, and the activity of cytotoxic T lymophocytes (CTL).

The immunomodulator of the present invention may be used for health promoting purposes. It is also effective against diseases or conditions such as tumor, cancer metastasis, viral diseases (e.g. cold, AIDS, and viral hepatitis), allergic diseases (e.g. pollinosis, allergic rhinitis, atopy, and asthma), autoimmune diseases (e.g. rheumatoid arthritis), inflammatory disease, and diabetes.

### Food additives, and foods and drinks that contain fucoidan or its hydrolysate and the immuno-stimulating material

If the combination of the present invention which comprises fucoidan or its hydrolysate and the immuno-stimulating material is to be used in foods or beverages, it is advantageously applied not only in food additives or in foods or beverages that contain it and which have the immunomodulatory activity but also in health foods that have those food additives added thereto and which have the immunomodulatory activity. These can be mixed with known sweeteners, acid flavors, vitamins and other ingredients to formulate products that suit the preferences of various users. The foods and beverages may be offered in various forms including tablets, capsules, soft drinks, tea beverages, energy drinks, dairy products (yoghurt and lactic acid bacteria beverages), seasonings, processed foods, desserts, confectioneries (e.g. gum, candy, and jelly), etc. The foods and beverages according to the present invention include physiologically functional foods (including FOSHUs, or foods for specified health use, and qualified FOSHUs) with an indication that states that they have the immunomodulatory activity either on the container or in an instruction. The site of such indication may be on the container itself or on an instruction label attached to it but these are not the sole examples. The container may be exemplified by, but is not limited to, glass bottles, cans, PET and other plastic bottles, and paper packs. Means of indication include, but are not limited to, printing, stamping, and sealing. The foods may be pet food and animal feeds.

### Pharmaceuticals comprising the combination of fucoidan or its hydrolysate and the immuno-stimulating material

If the composition of the present invention is to be used as a pharmaceutical, it may be applied as, for example, an immunomodulator, an immuno-stimulating agent, an anti-allergic agent or an immunosuppressant. Therefore, in yet another aspect, the present invention provides a pharmaceutical composition that comprises fucoidan or its hydrolysate and the immuno-stimulating material and which displays an immunomodulatory activity, an immuno-stimulating action, an anti-allergic action and/or an immunosuppressing action.

The pharmaceutical composition may be formulated in various dosage forms by mixing the drug with known auxiliary agents that are commonly used in the pharmaceutical formulation technology and which may be exemplified by diluents, carriers, excipients, binders, disintegrants, lubricants, flavoring agents, solvent promoters, suspending agents, coating agents, etc. Exemplary dosage forms include, but are not limited to, tablets, capsules, granules, powders, solutions, syrups, suppositories, creams, ointments, emulsions, cataplasms, injections, etc. The pharmaceuticals of the present invention may be administered through various routes including, but not limited to, oral administration, rectal administration, and through the intestines.

### Cosmetics comprising the combination of fucoidan or its hydrolysate and the immuno-stimulating material

The combination of the present invention may also be used to manufacture cosmetics having the immunomodulatory activity.

Cosmetics comprising the combination of the present invention include, but are not limited to, facial, skin or hair creams, lotions, gels, mousse, shampoos, and rinse.

### Combination with other ingredients

The composition comprising the combination of the present invention may be applied on its own in foods or beverages, pharmaceutical compositions, or in cosmetics. If desired, it may be applied together with other foodstuffs or substances that have the immunomodulatory activity or the immuno-stimulating action. Examples of such foodstuffs or substances include lactic acid bacteria, mushrooms, fucoidan, etc.

Aside from the active ingredients mentioned above, the food/beverage and composition of the present invention may incorporate common ingredients, such as carriers, diluents, excipients or additives, in accordance with specific embodiments. The carriers, diluents and excipients that may be used are not limited to any particular types as long as they do not interfere with the physiological activities of fucoidan or its hydrolysate and the immuno-stimulating material. Suitable examples include: sugars such as sucrose, glucose, fructose, maltose, trehalose, lactose, starch, glucose syrup, and high fructose syrup; alcohols such as ethanol, propylene glycol, and glycerin; sugar alcohols such as sorbitol, mannitol, erythritol, lactitol, xylitol, maltitol, reduced palatinose, and reduced starch hydrolysate; solvents such as triacetin; polysaccharides such as gum arabic, carageenan, xanthan gum, guar gum, gellan gum, and pectin; and water. Exemplary additives include aids such as chelating agents, flavors, spice extracts, and antiseptics. The diluents, carriers, additives, etc. may be incorporated to the extent that will not impair the intended advantages of the present invention.

The amounts in which fucoidan or its hydrolysate and the immuno-stimulating material are incorporated in foods or beverages, pharmaceutical compositions, and in cosmetics are not limited in any particular way and may be selected as appropriate considering the other ingredients they are to be combined with. However, the sum of the amounts of fucoidan or its hydrolysate and the immuno-stimulating material, when they are to be incorporated in foods or beverages on in pharmaceutical compositions, generally ranges from 0.01 g to 10 g/day, preferably from 0.05 to 1 g/day, most preferably from 0.05 g to 0.5 g/day, for an individual weighing 60 kg; when they are to be incorporated in cosmetics, the sum of their amounts generally ranges from 0.01 to 20 wt%, preferably from 0.05 to 15 wt%.

Fucoidan or its hydrolysate and oligosaccharides of the present invention may be prepared either as purified extracts or as synthetic products, which may be applied individually in foods or beverages, pharmaceutical compositions or in cosmetics.

It should be noted here that the present invention is by no means limited to the foregoing embodiments and that various modifications can be made within the scopes defined in the accompanying claims; it should also be noted that the technical means disclosed in different embodiments may be combined appropriately to work out other embodiments, which are also encompassed within the technical scope of the present invention.

### EXAMPLES

The present invention is described below in greater detail with reference to working examples but it should be understood that the scope of the present invention is by no means limited to those examples.

Unless otherwise noted, a nuclear magnetic resonance apparatus of Model ECA-600 (JEOL) was used in the following examples in order to perform NMR analysis. Heavy water (D₂O) was used as a solvent for measurement. The mode in which the constituent sugars were bound was investigated by 2D-NMR.

### EXAMPLE 1

### Preparation of Fucoidan Fractions

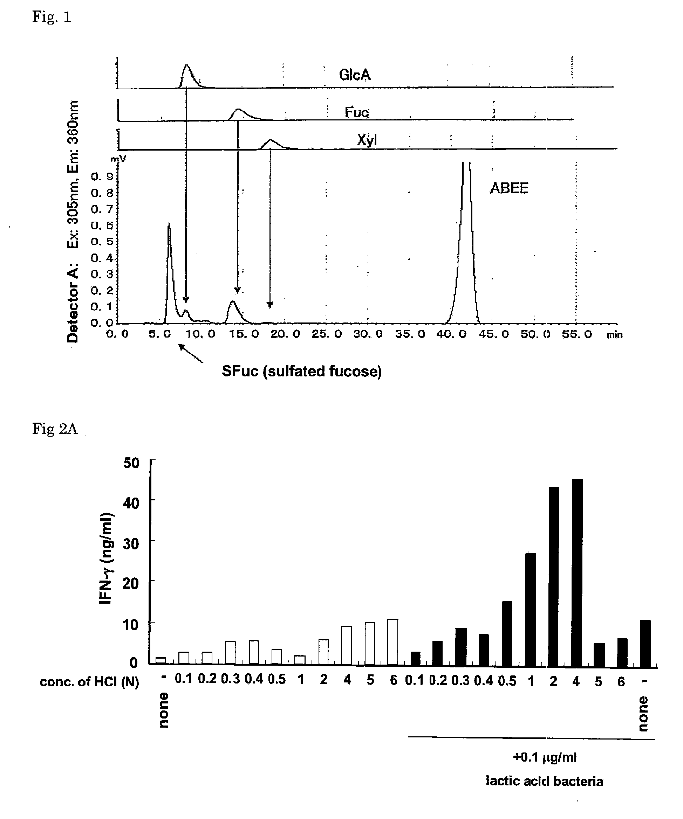

To 100 g of an algal body of Okinawa Nemacystus decipiens, 1000 ml of distilled water was added and conducted extraction at 100°C for 1 hour. The resulting extract was cooled, filtered by suction, electrodialyzed (desalted) and freeze-dried to give fucoidan fractions in an amount of 2 g. The fucoidan was subjected to 1-hr hydrolysis in an aqueous solution at 100°C containing 2 N H₂SO₄ and the resulting aqueous solution was neutralized with 2 N NaOH, and labeled fluorescently with ABEE to prepare a sample for analysis of monosaccharides. It was verified that the constituent sugars in the sample were SFuc (sulfated fucose), GlcA (glucuronic acid), Fuc (fucose) and Xyl (xylose) which were present at a ratio of 49.3:4.9:12.1:1 (see Fig. 1).
Column: Cosmosil C18 AR-II (4.6 mmφ x 250 mm)
Mobile phase: 0.2 M potassium borate buffer containing 10% acetonitrile
Flow rate: 1.0 ml/min
Temperature: 45°C
Detection: Fluorescence detector (Shimadzu Corporation), Ex: 305 nm; Em: 360 nm

### EXAMPLE 2

### Production of Fucoidan Hydrolysates and Measurmeent of Immune Immunomodulatory activity

Fucoidan hydrolysates were prepared under the varying conditions shown below and combined with lactic acid bacteria to evaluate the immunomodulatory activity.

### The effect of acid concentration for hydrolysis on the immunomodulatory activity

The fucoidan prepared in Example 1 was hydrolyzed with 0.1, 0.2, 0.3, 0.4, 0.5, 1.0, 2.0, 4.0, 5.0 or 6.0 N HCl (80°C) for one hour; thereafter, 0.1, 0.2, 0.3, 0.4, 0.5, 1.0, 2.0, 4.0, 5.0 or 6.0 N NaOH were added to the respective samples to neutralize them; the neutralized samples were desalted by electrodialysis and freeze-dried to prepare fucoidan hydrolysates. Each of the fucoidan hydrolysates was added at a concentration of 100 µg/ml to splenocytes (5x10⁶ cells/ml) isolated from C57BL/6 mice (8-wk old, male, CHARLES RIVER LABORATORIES JAPAN, INC.) Thirty minutes later, 0.1 µg/ml of lactic acid bacteria (Lactobacillus pentosus: FERM ABP-10028) was added and culture was performed for 24 hours; the supernatant of the culture was recovered and the production of IFN-γ was quantified using an ELISA kit (OptEIA, BD Pharmingen). The results are shown in Fig. 2A. In order to study the effect of using the fucoidan hydrolysate alone, the similar procedure was taken without using the lactic acid bacteria (open bars). As three controls, nothing was added (an open bar, "none"), and only the lactic acid bacteria were added (a closed bar, "none"). When the products obtained by hydrolysis were used in combination with the lactic acid bacteria, the production of IFN-γ was synergistically increased compared with the results obtained by using them individually. This synergism was recognized in the products obtained by hydrolysis with 0.5-4.0 N HCl.

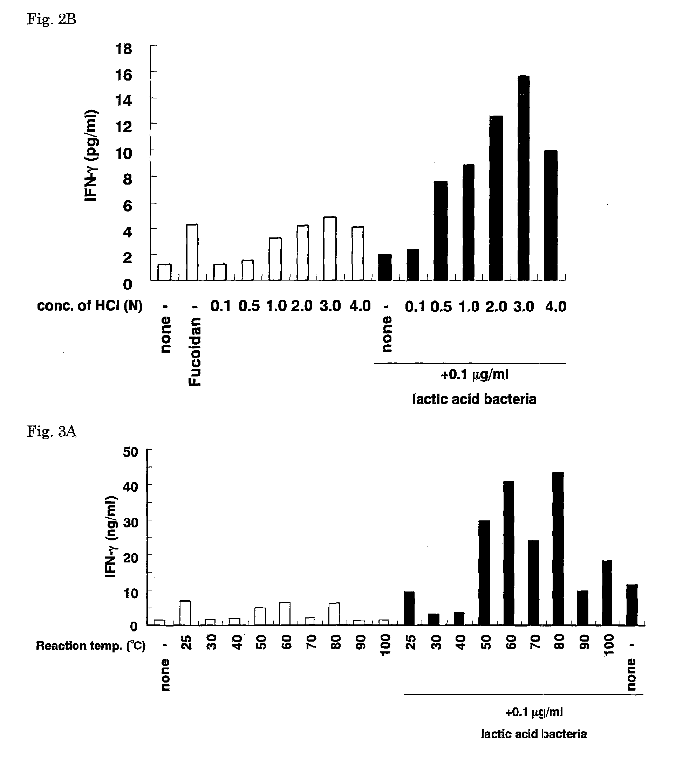

Further, the same operation was performed using fucoidan (before hydrolysis) alone, and the effect of fucoidan was compared with those of other samples (Fig. 2B). The hydrolysates obtained by hydrolysis with 0.5-4.0 N HCl (Fig. 2B, open bars) only showed activity comparable to the fucoidan before hydrolysis (Fig 2B, "fucoidan"). However, the results reveal that the addition of the lactic acid bacteria to the hydrolysates gave a greater activity than could be achieved by fucoidan alone (see Fig. 2B, closed bars).

### The effect of reaction temperature

Fucoidan was hydrolyzed with 1 N HCl at 25, 30, 40, 50, 60, 70, 80, 90 or 100°C for one hour; thereafter, 1.0 N NaOH was added to the respective samples to neutralize them; the neutralized samples were desalted by electrodialysis and freeze-dried to prepare fucoidan hydrolysates. Each of the fucoidan hydrolysates was added at a concentration of 100 µg/ml to splenocytes (5x10⁶ cells/ml) isolated from C57BL/6 mice (8-wk old, male, CHARLES RIVER LABORATORIES JAPAN, INC.). Thirty minutes later, 0.1 µg/ml of lactic acid bacteria (Lactobacillus pentosus: FERM ABP-10028) were added and culture was performed for 24 hours; the supernatant of the culture was recovered and the production of IFN-γ was quantified using an ELISA kit (OptEIA, BD Pharmingen). The results are shown in Fig. 3A. In order to study the effect of using the fucoidan hydrolysate alone, the similar procedure was taken without using the lactic acid bacteria (open bars). As three controls, nothing was added (an open bar, "none"), and only the lactic acid bacteria were added (a closed bar, "none"). When the fucoidan hydrolysates obtained by hydrolysis with HCl were used in combination with the lactic acid bacteria, the production of IFN-γ was synergistically increased compared with the results obtained by using them individually. This synergism was recognized in all the products obtained by hydrolysis at 25-100°C, with particularly good results from the hydrolysis effected at 30-90 °C (preferably, 50-80°C).

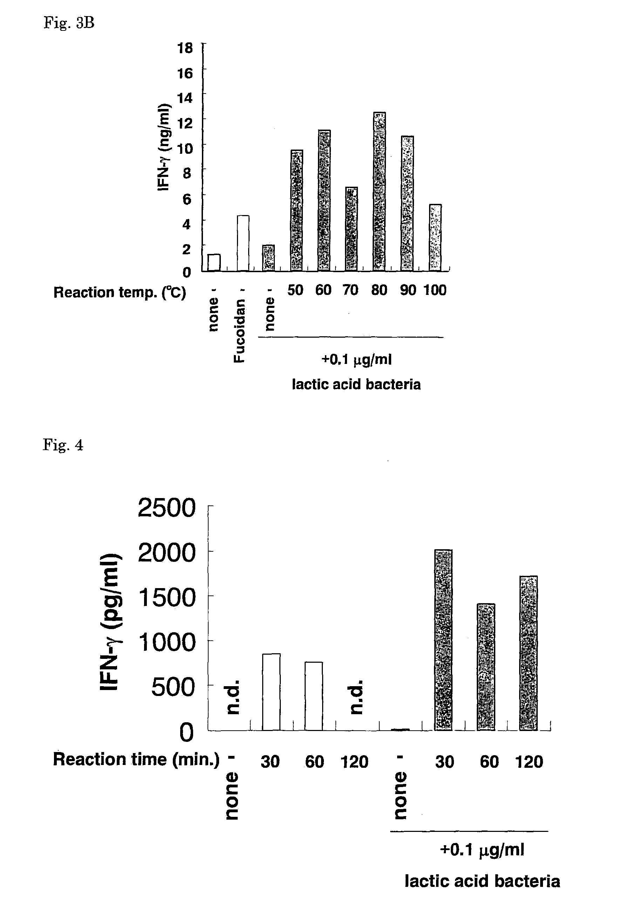

Further, the experiment mentioned above was conducted for fucoidan (before hydrolysis) alone, and the effect was compared with those of the present invention. As a result, it became clear that the products obtained by hydrolysis with HCl at 50-100°C shows an activity at least comparable to the fucoidan before hydrolysis, when the hydrolysis products are combined with lactic acid bacteria (see Fig. 3B).

### The effect of reaction time

Fucoidan was hydrolyzed with 1 N HCl (80°C) for 30, 60 or 120 minutes; thereafter, 1.0 N NaOH was added to the respective samples to neutralize them; the neutralized samples were desalted by electrodialysis and freeze-dried to prepare fucoidan hydrolysates. Each of the fucoidan hydrolysates was added at a concentration of 100 µg/ml to splenocytes (5x10⁶ cells/ml) isolated from C57BL/6 mice (8-wk old, male, CHARLES RIVER LABORATORIES JAPAN, INC.). Thirty minutes later, 0.1 µg/ml of lactic acid bacteria (Lactobacillus pentosus: FERM ABP-10028) was added and culture was performed for 24 hours; the supernatant of the culture was recovered and the production of IFN-γ was quantified using an ELISA kit (OptEIA, BD Pharmingen). The results are shown in Fig. 4. In order to study the effect of using the fucoidan hydrolysate alone, the similar procedure was taken without using the lactic acid bacteria. As two controls, nothing was added and only the lactic acid bacteria were added. With all the products obtained by hydrolysis for 30-120 minutes, the combination with the lactic acid bacteria allowed the production of IFN-γ to be synergistically increased compared with the results obtained by using them individually.

The above results show that the fucoidan hydrolysate, if used in combination with lactic acid bacteria, exhibits a synergistically enhanced immunomodulatory or immuno-stimulating action.

### EXAMPLE 3

### The Immunomodulatory Actin of Purified Fucoidan Hydrolysates

To 1 g of fucoidan, 100 ml of 2 N HCl was added and hydrolysis was performed at 80°C for one hour, then followed by neutralization with 1 N NaOH. The resulting reaction mixture was desalted by gel filtration (BIOGEL P-6 (Bio-Rad)) and then freeze-dried to give a fraction containing a mixture of fucoidan oligosaccharides (oligosaccharide fraction). The thus obtained mixture of fucoidan oligosaccharides was subjected to chromatography using a formic acid activated anion-exchange resin (TOSOH CORPORATION). As a result, the oligosaccharide fraction was separated into two fractions, Fr. B, that resulted from elution with water and which contained oligosaccharides free of sulfuric acid groups, and a sulfated saccharide fraction, that resulted from elution with 2 N HCl and which contained oligosaccharides rich in sulfuric acid groups. The sulfated saccharide fraction was subjected to gel filtration (BIOGEL P-6) so that it was separated into Fr. A with a molecular weight less than 500 and Fr. C of 500 or more. Each of the thus purified fucoidan hydrolysates was added at a concentration of 100 µg/ml to splenocytes (5×10⁶ cells/ml) isolated from C57BL/6 mice (8-wk old, male, CHARLES RIVER LABORATORIES JAPAN, INC.). Thirty minutes later, 0.1 µg/ml of lactic acid bacteria (Lactobacillus pentosus: FERM ABP-10028) was added and culture was performed for 24 hours; the supernatant of the culture was recovered and the production of IFN-γ was quantified using an ELISA kit (OptEIA, BD Pharmingen). The results are shown in Table 1 below. As a result, fucoidan showed the activity when it was used at a lower concentration, but the activity was lost as its concentration was increased; on the other hand, all of the hydrolysates (the oligosaccharide fraction, Fr. A, Fr. B, Fr. C, and the sulfated saccharide fraction) had their immuno-stimulating ability increased in a dose-dependent manner to exhibit stronger activity than fucoidan. It was also found that the oligosaccharides became more active upon purification. The controls were monosaccharides (fucose and sulfated fucose), and the widely used prebiotics xylooligosaccharides, and they had none of the activities described above.

**Table 1**

| | +0.1 µg/ml lactic acid bacteria | | | |
|---|---|---|---|---|
| | 3 | 10 | 30 | 100 |
| | (µg/ml) | | | |
| fucoidan | 9.8 | 10.2 | 5.2 | n.d. |
| fucose | 1.2 | 1.9 | - | 2.9 |
| sulfated fucose | 1.3 | 0.5 | - | n.d. |
| oligosaccharide fraction | n.d. | n.d. | 5.5 | 9.6 |
| Fr.A | 10.5 | 17.2 | 21.2 | 15.8 |
| Fr.B | 4.6 | 6.7 | 11.0 | 21.2 |
| Fr.C | 5.0 | 11.5 | 19.3 | 21.0 |
| sulfated saccharide fraction | 0.0 | 19.8 | - | 21.3 |
| Xylooligosaccharides | n.d. | n.d. | - | n.d. |
| Lactic acid bacteria | 0 | 0.1 µg/ml | | |
| | n.d. | n. d. | | |

### EXAMPLE 4

### Production of Fucoidan Oligosaccharides

### a) Hydrolysis of fucoidan and separation of oligosaccharide mixture

To 1 g of the fucoidan fraction obtained in Example 1, 100 ml of 2 N HCl was added and hydrolysis was performed at 50-100°C for one hour, then followed by neutralization with 1 N NaOH. The resulting reaction mixture was desalted by gel filtration (BIOGEL P-6 (Bio-Rad)) and then freeze-dried to give a mixture of fucoidan oligosaccharides in an amount of 895 mg. The thus obtained mixture of fucoidan oligosaccharides was subjected to chromatography using a formic acid activated anion-exchange resin (TOSOH CORPORATION). As a result, the mixture of oligosaccharides was separated into 280 mg of a fraction (consisting of neutral and acidic saccharides) that resulted from elution with water and which contained oligosaccharides free of sulfuric acid groups, and 425 mg of a fraction (a sulfated saccharide fraction) that resulted from elution with 2 N HCl and which contained oligosaccharides rich in sulfuric acid groups.

### b) Isolation of compounds (I) and (II)

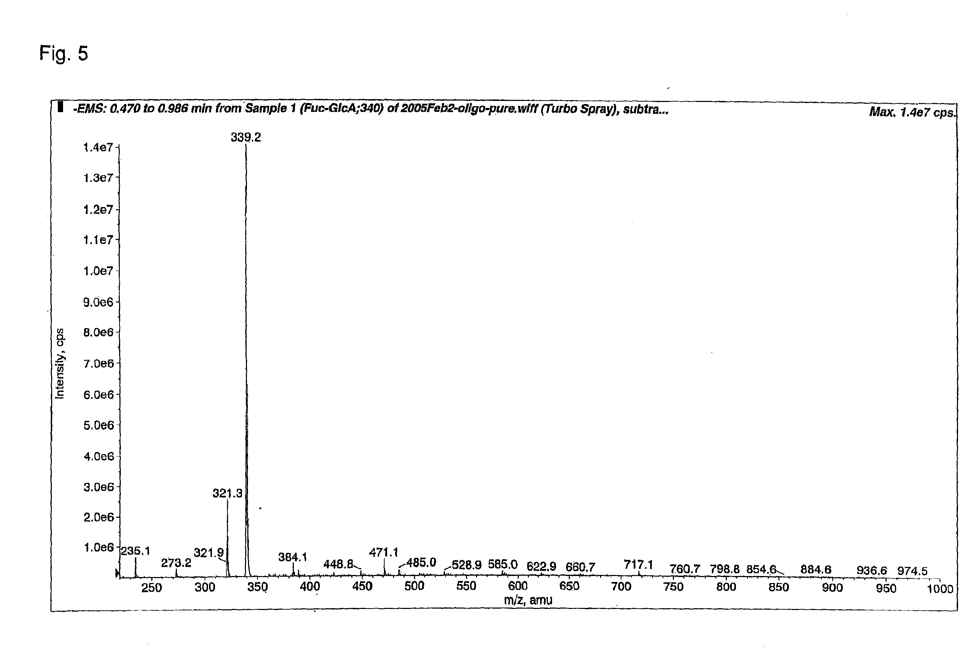

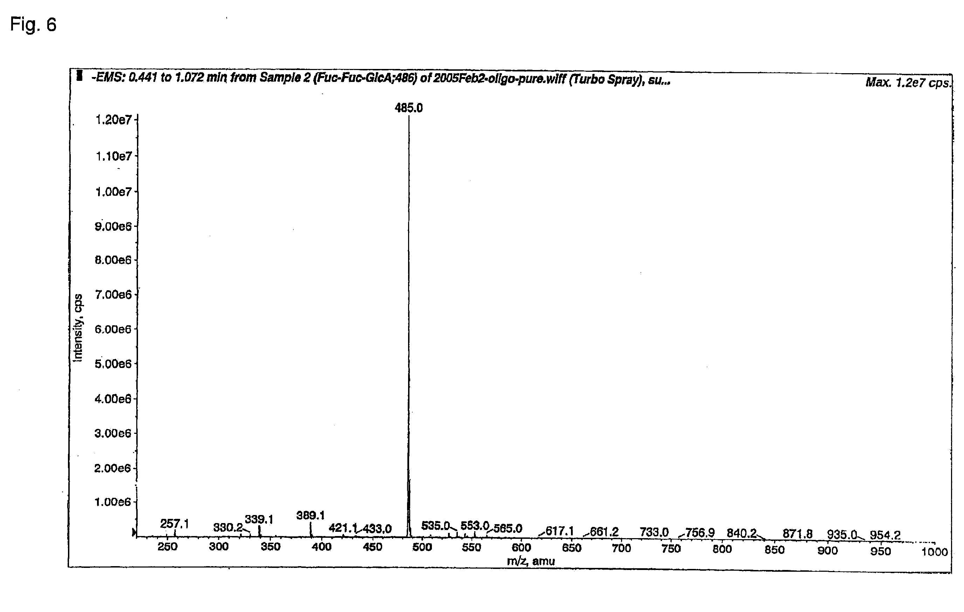

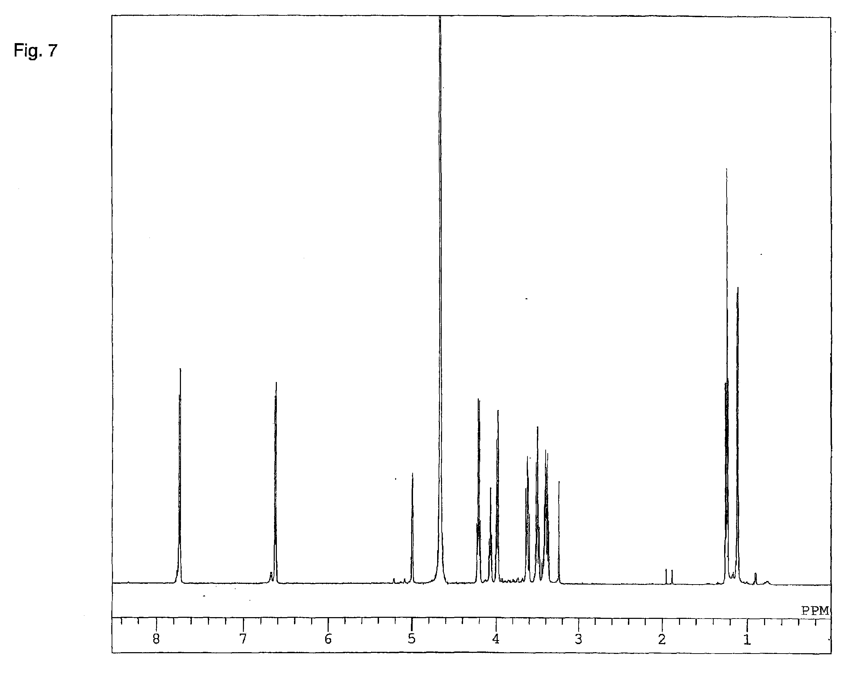

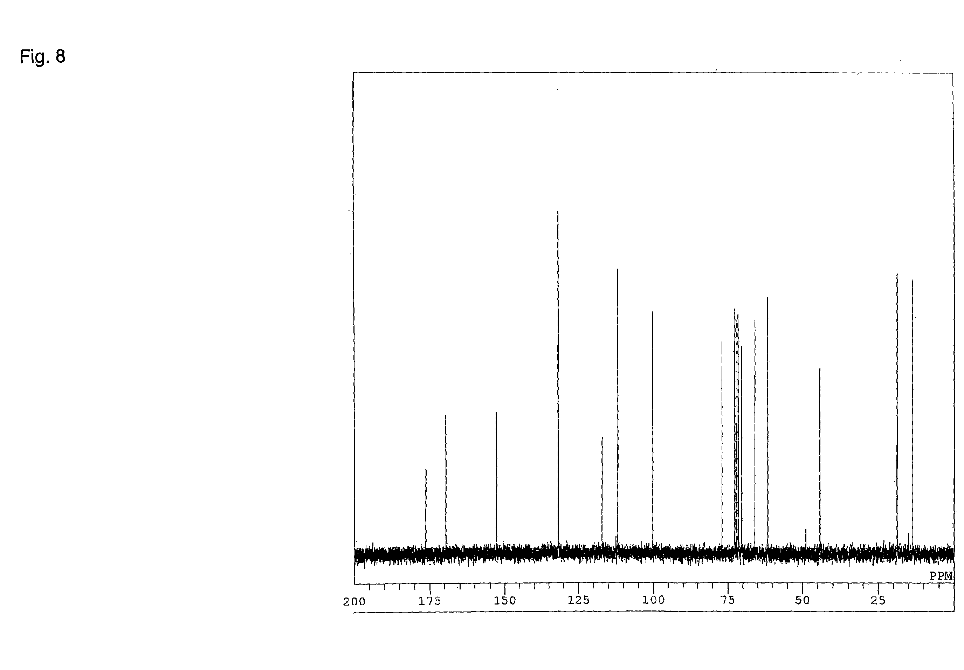

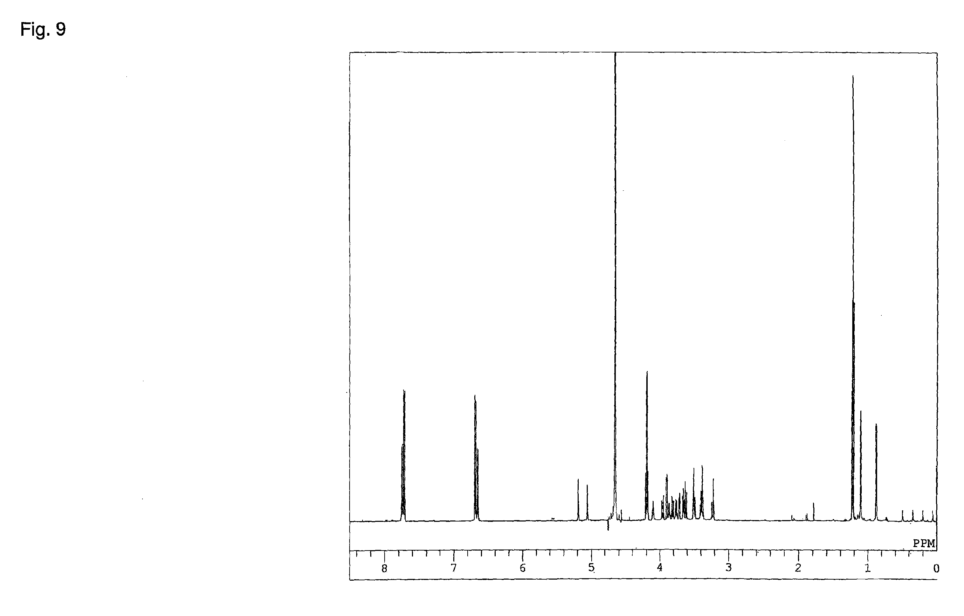

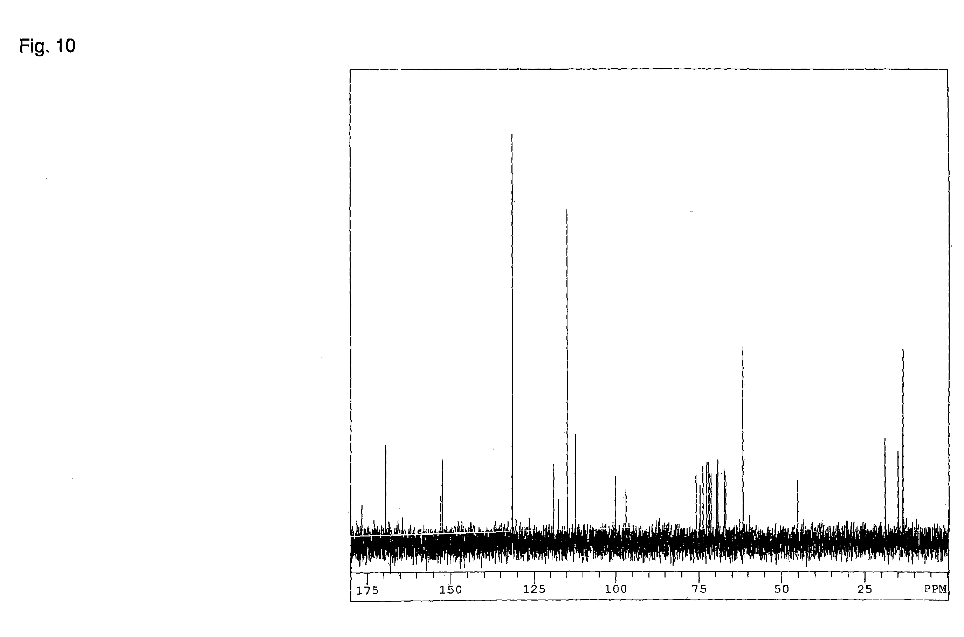

A hundred milligrams of the neutral and acidic saccharide fraction obtained in a) was subjected to gel filtration (BIOGEL P-4 (Bio-Rad); eluting solvent: aqueous solution of 0.2 M potassium borate (K₂B₄O₇)), whereupon a disaccharide having a molecular weight of 340 (compound (I), see Fig. 5) and a trisaccharide having a molecular weight of 486 (compound (II), see Fig. 6) separated out from the fraction. The molecular weights of the two compounds were determined by FAB-MS (compound (I)[M-H]⁻: 339.2; compound (II) [M-H]⁻: 485.0). To 5 mg of each of these compounds, 1 ml of water, 1.6 g of ABEE (4-aminobenzoic acid ethyl ester), 350 mg of NaBH₃CN (sodium cyanoborohydride), 3.5 ml of methanol and 410 µl of acetic acid were added and the mixture was stirred at 65°C for 4 hours. The reaction mixture was partitioned with chloroform and water to thereby acquire about 7-9 mg of the fluorescently labeled di- and trisaccharide. The thus labeled oligosaccharides were subjected to ¹H-NMR and ¹³C-NMR; the resulting charts are shown in Figs. 7-10 and the results of their analyses are shown in Tables 2 and 3 below. As it turned out, the disaccharide having a molecular weight of 340 was α-D-GlcA-(1→2)-L-Fuc represented by formula (I), and the trisaccharide having a molecular weight of 486 was α-D-GlcA-(1→2)-α-L-Fuc-(1→3)-L-Fuc represented by formula (II).

**Table 2: Results of ¹H-NMR and ¹³C-NMR analyses of the fluorescently labeled compound (I)**

| Position | ¹H -NMR (D₂O) δ | ¹³C -NMR (D₂O) δ |
|---|---|---|
| ABEE-1 | - | 117.0 |
| -2,6 | 7.74(2H, d, J= 7.6Hz) | 131.7 |
| -3,5 | 6.63(2H, d, J= 7.6Hz) | 111.8 |
| -4 | - | 152.8 |
| -C=O | - | 169.6 |
| -CH₂ | 4.21(2H, q, J=14.3, 7.0, 1.5Hz) | 61.6 |
| -CH₃ | 1.24(3H, td, J=7.1, 1.6Hz) | 13.6 |
| Fuc-1(CH₂) | 3.41(2H, m) | 44.3 |
| -2 | 4.07(1H, t, J= 6.4Hz) | 77.1 |
| -3 | 3.56(1H, m) | 72.7 |
| -4 | 3.51(1H, m) | 72.8 |
| -5 | 3.99(1H, d, J= 8.6Hz) | 66.0 |
| -CH₃ | 1.12(3H, d, J= 6.5Hz) | 18.8 |
| GlcA-1 | 5.00(1H, d, J= 3.4Hz) | 100.2 |
| -2 | 3.48(1H, m) | 71.6 |
| -3 | 3.63(1H, m) | 70.5 |
| -4 | 3.38(1H, m) | 72.0 |
| -5 | 4.00(1H, t, J= 6.4Hz) | 72.4 |
| -COOH | - | 176.3 |

**Table 3: Results of ¹H-NMR and ¹³C-NMR analyses of the fluorescently labeled compound (II)**

| Position | ¹H -NMR (D₂O) δ | ¹³C -NMR (D₂O) δ |
|---|---|---|
| ABEE-1 | - | 117.6 |
| -2,6 | 7.746 (2H, d, J = 8.9 Hz) | 131.6 |
| -3,5 | 6.655 (2H, d, J = 8.6 Hz) | 112.4 |
| -4 | - | 152.9 |
| -C=O | - | 169.4 |
| -CH₂ | 4.191 (2H, q, J = 7.1 Hz) | 61.7 |
| -CH₃ | 1.220 (3H, t, J = 7.0 Hz) | 13.6 |
| Fuc-1(CH₂) | 3.396 (1H, q, *J* = 6.2 Hz) 3.223 (1H, q, *J* = 6.9 Hz) | 45.2 |
| -2 | 4.115-4.089 (1H, m) | 69.8 |
| -3 | 3.713 (1H, q, *J* = 2.4 Hz) | 75.9 |
| -4 | 3.654 (1H, t, *J* = 4.6 Hz) | 73.9 |
| -5 | 3.866 (1H, dt, *J* = 12.3, 5.2 Hz) | 67.0 |
| -CH₃ | 1.103 (3H, d, J = 6.5 Hz) | 18.9 |
| Fuc-1 | 5.061 (1H, d, J = 4.1 Hz) | 97.0 |
| -2 | 3.812 (1H, dd, J = 10.3, 3.8 Hz) | 74.9 |
| -3 | 3.960 (1H, dd, J = 10.7, 3.4 Hz) | 69.4 |
| -4 | 3.506 (1H, t, J = 4.1.Hz) | 72.2 |
| -5 | 3.760 (1H, q, J = 6.5 Hz) | 67.4 |
| -CH₃ | 0.876 (3H, d, J = 6.5 Hz) | 15.1 |
| GlcA-1 | 5.187 (1H, d, J = 3.8 Hz) | 100.3 |
| -2 | 3.495 (1H, dd, J = 10.0, 4.1 Hz) | 71.5 |
| -3 | 3.624 (1H, t, J = 9.5 Hz) | 72.9 |
| -4 | 3.380 (1H, t, J = 9.6 Hz) | 72.0 |
| -5 | 3.903 (1H, d, J = 10.0 Hz) | 72.8 |
| -COOH | - | 176.6 |

### c) Production of compounds (III) - (XI)

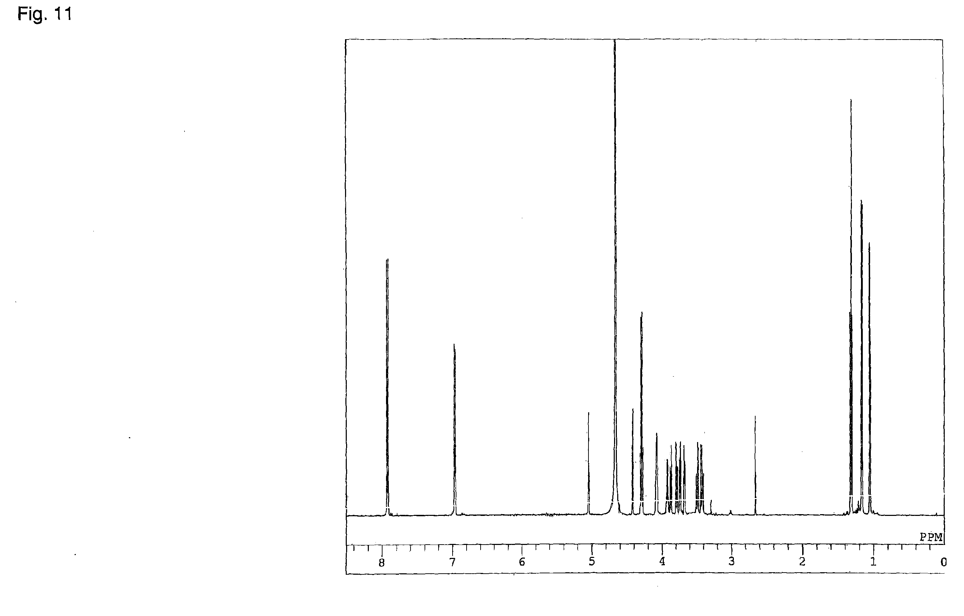

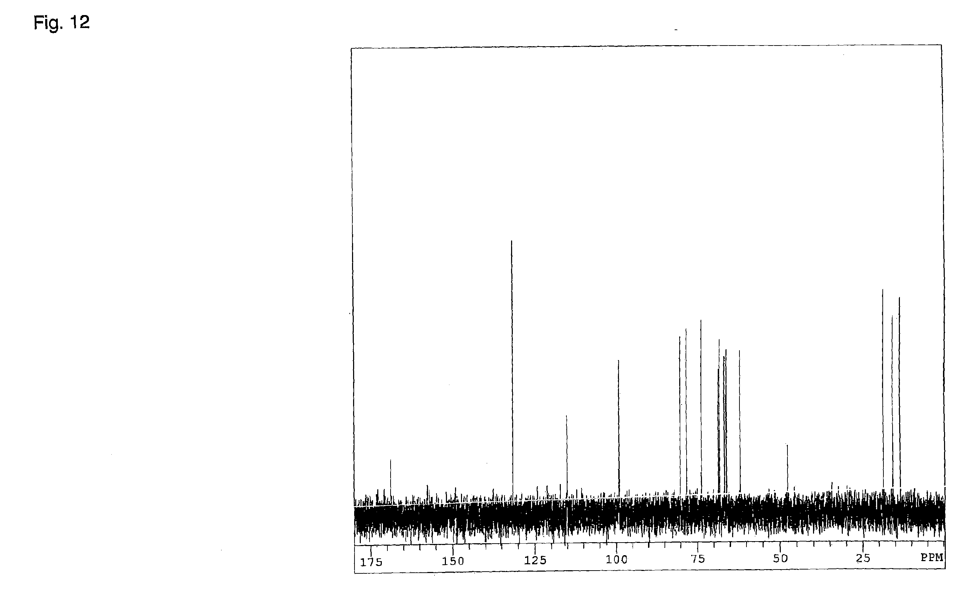

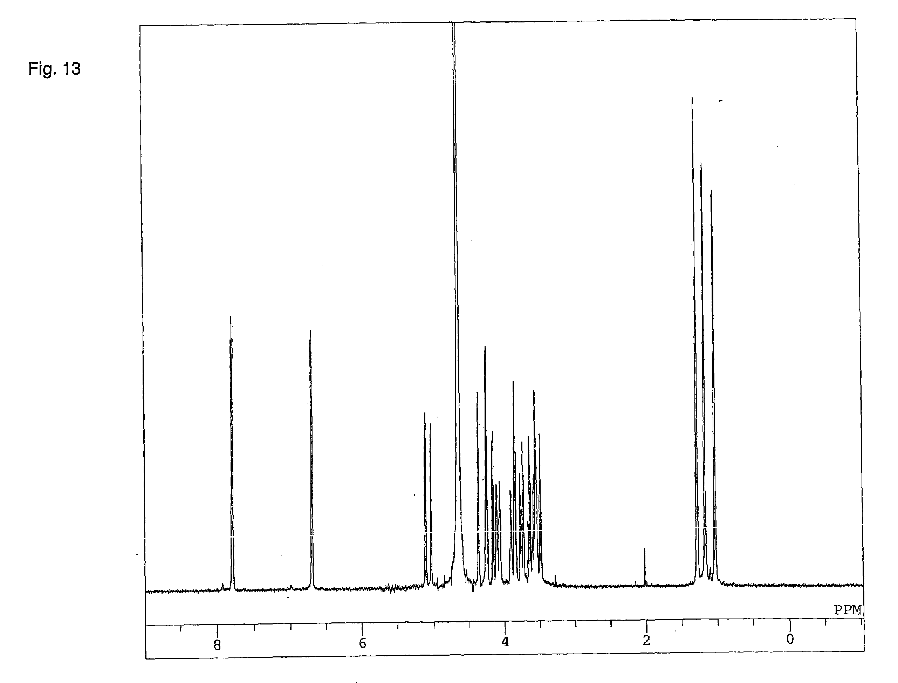

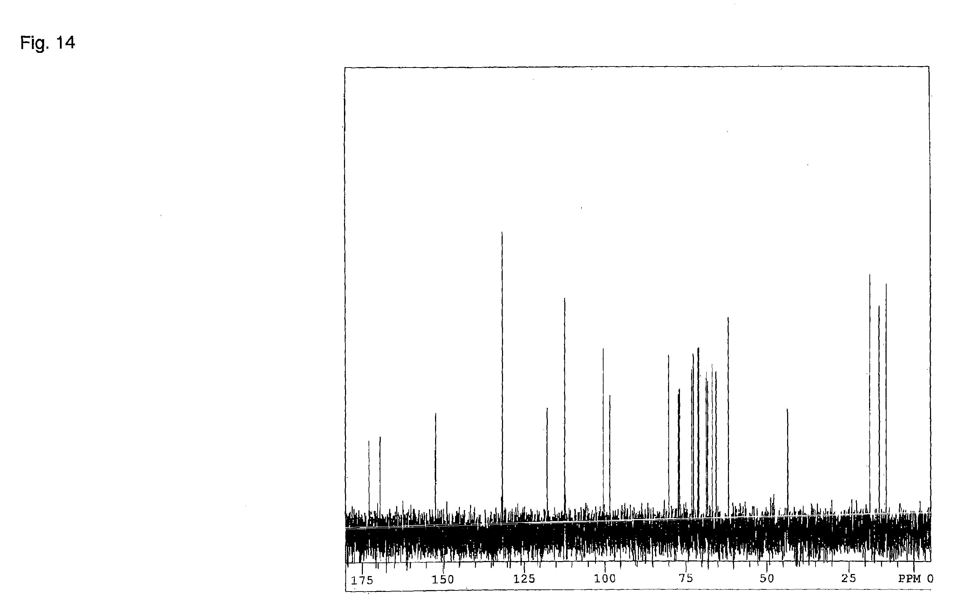

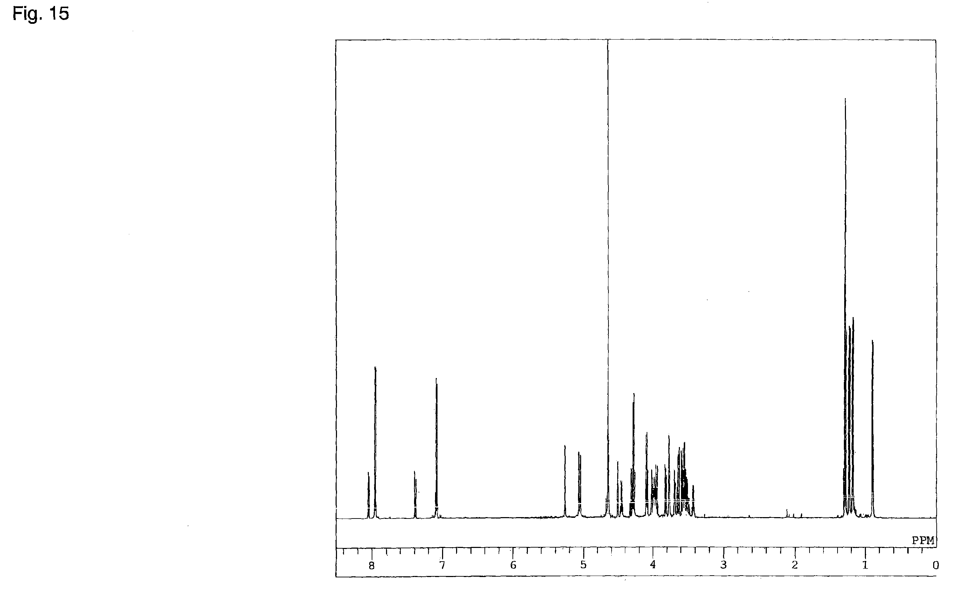

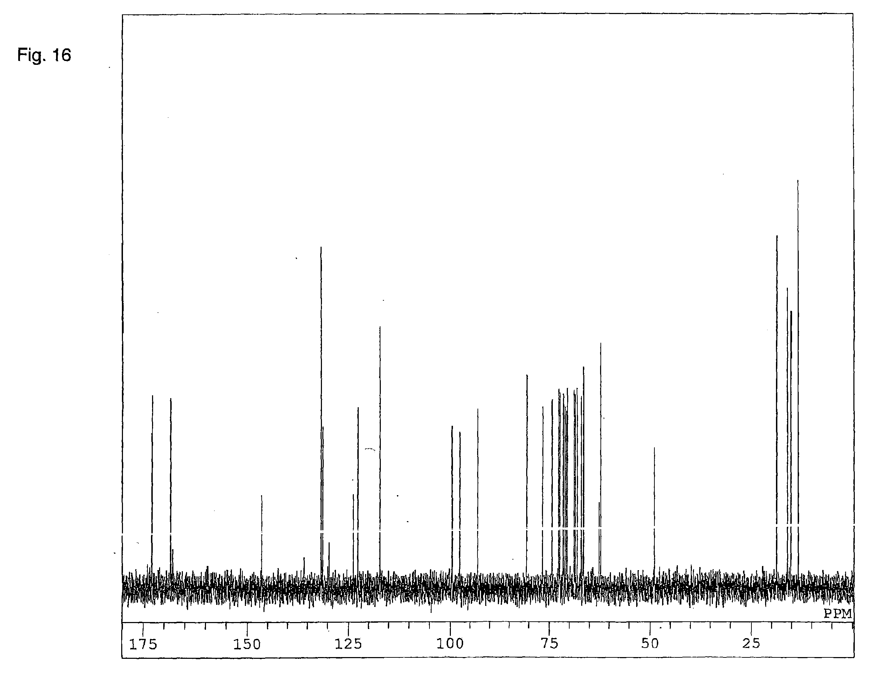

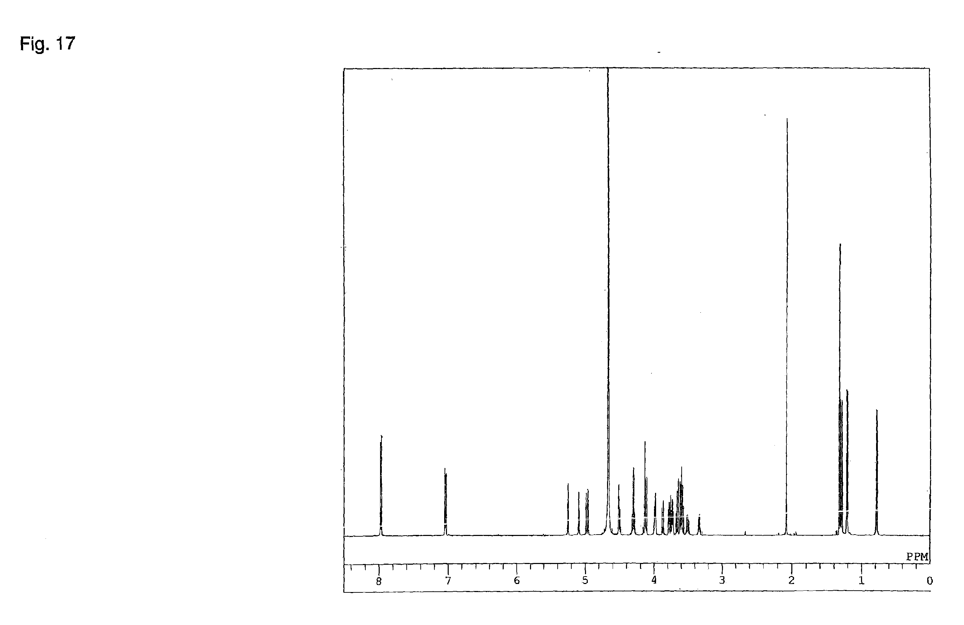

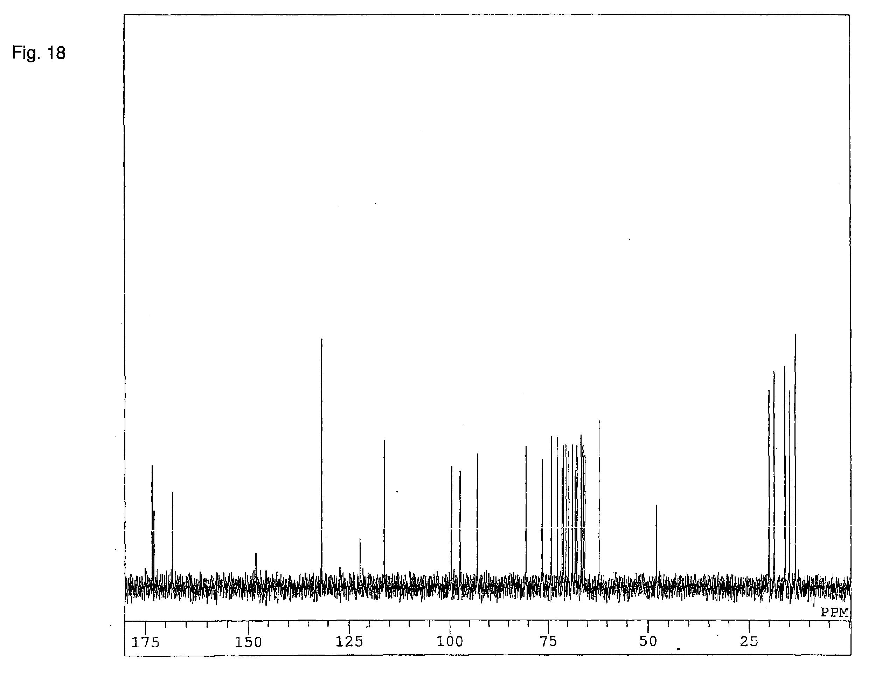

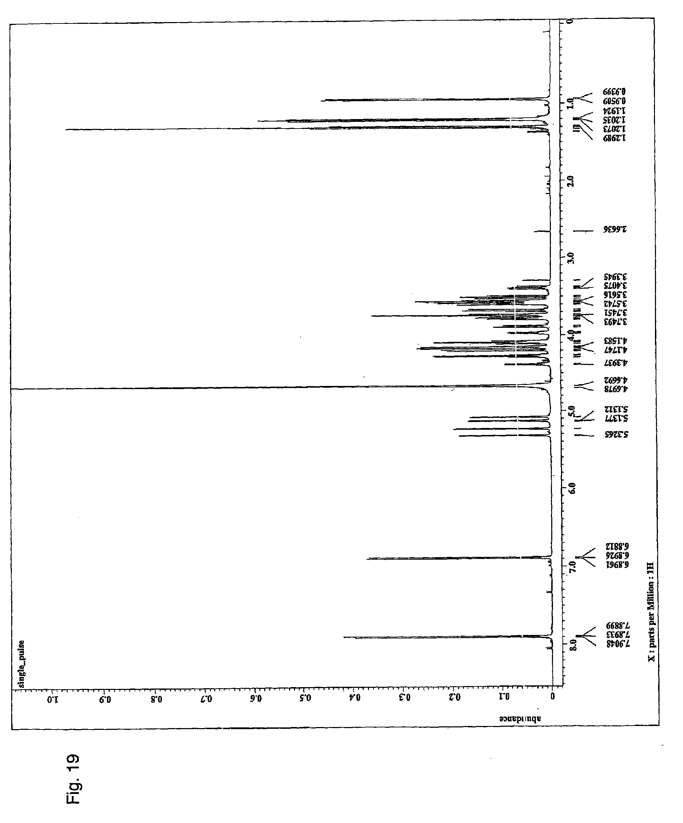

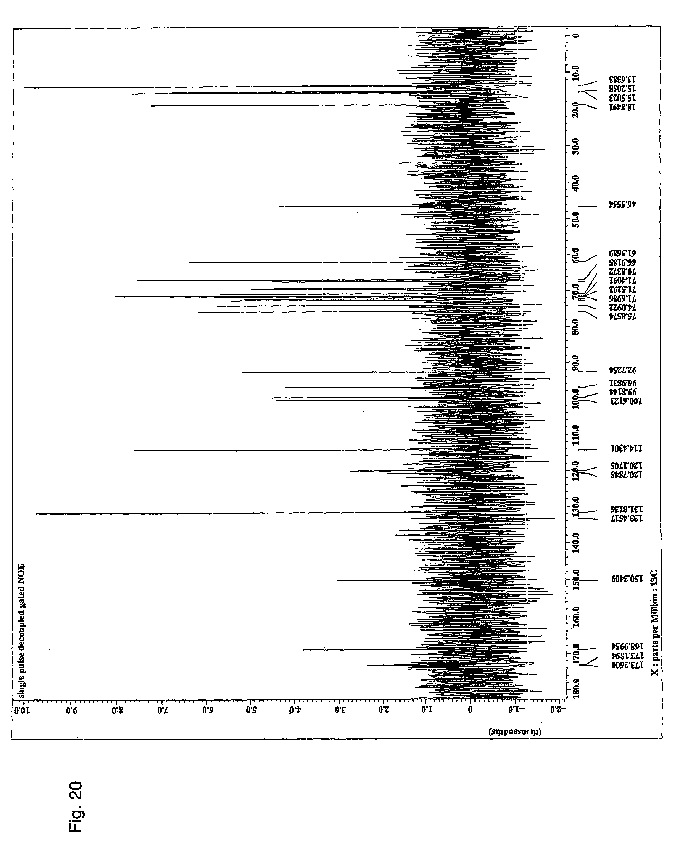

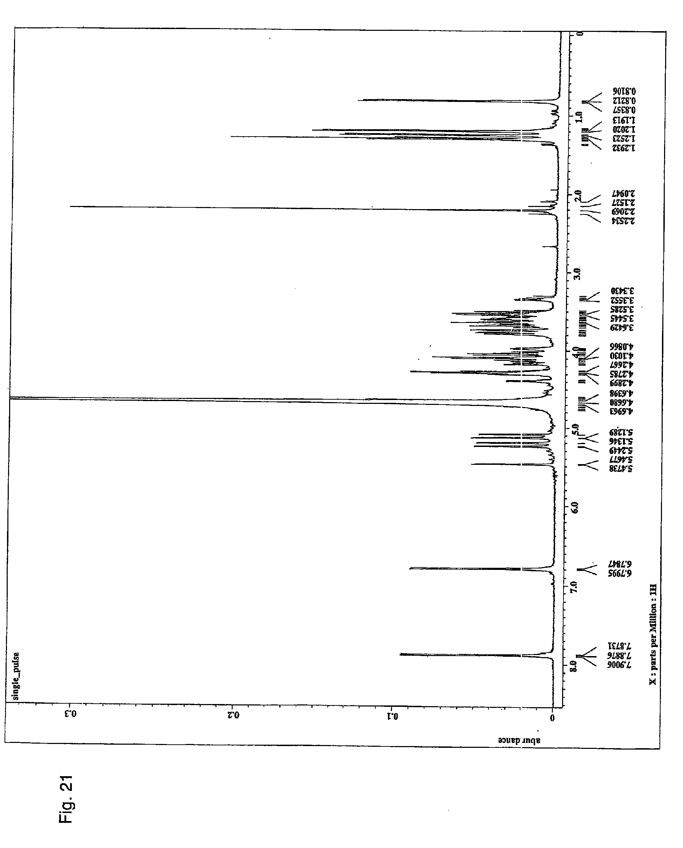

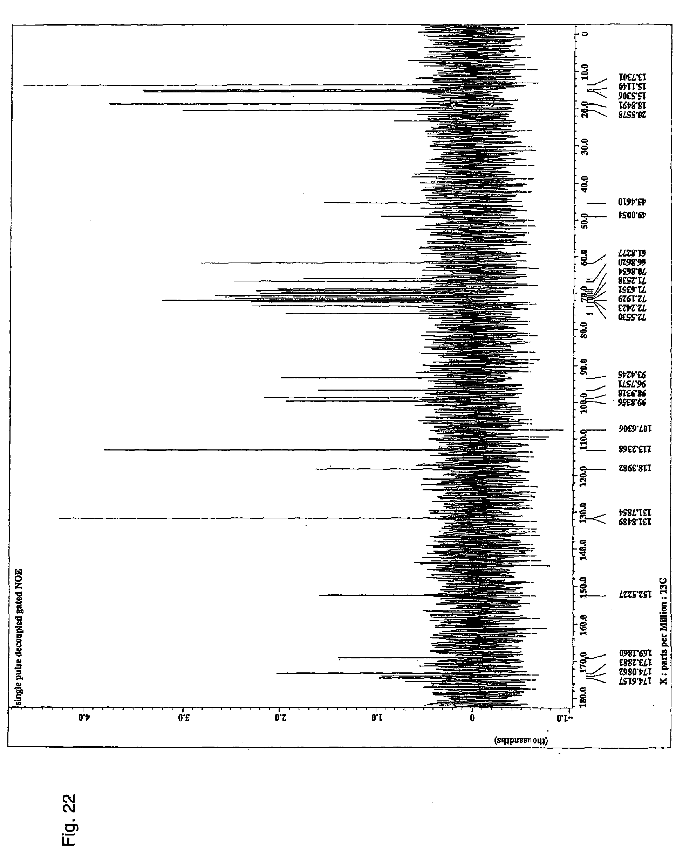

In the next step, the sulfated saccharide fraction was subjected to gel filtration (BIOGEL P-6 (Bio-Rad)) for desalting. To 100 mg of the resulting sulfated saccharide fraction, 1 ml of water, 1.6 g of ABEE (4-aminobenzoic acid ethyl ester), 350 mg of NaBH₃CN (sodium cyanoborohydride), 3.5 ml of methanol and 410 µl of acetic acid were added and the mixture was stirred at 65°C for 4 hours. The reaction mixture was dried under vacuum and partitioned with chloroform and water, and the aqueous phase was treated on a reverse-phase column (carrier: Lichroprep RP-8 (25-40 µm) (Merck), φ10 x 220 mm; solvent conditions: 5% CH₃CN/0.1% TFA (100 ml), 8% CH₃CN/0.1% TFA (100 ml); 15%. CH₃CN/0.1% TFA (100 ml), and 20% CH₃CN/0.1% TFA (100 ml)) to thereby yield a mixture of labeled oligosaccharides. The resulting fluorescently labeled compounds were subjected to HPLC (column: cosmosil 5C18-AR-II, φ10.0 x 250 mm; solvent conditions: 12.5% CH₃CN/0.1% TFA (5 min) and 12.5-27.5% CH₃CN/0.1% TFA (50 min); flow rate: 3 ml/min) with a mixture of acetonitrile and a 0.1% TFA aqueous solution being flowed as an eluting solution at a density gradient of 5-30%, whereupon six labeled fucoidan oligosaccharides having sulfuric acid groups were separated from the mixture; they respectively had molecular weights of 539, 715, 861, 903, 957 and 999 (as determined by ESI-MS). The labeled fucoidan oligosaccharides were then measured for NMR spectra and their results were analyzed. The ¹H-NMR and ¹³C-NMR charts of the labeled oligosaccharides are shown in Figs. 11-22 and the results of their analyses are shown in Tables 4 - 7. From those results, the compounds having molecular weights of 539, 715, 861, 903, 957 and 999 were found to be labeled forms of compounds (III), (V), (VI), (VII), (VIII) and (IX), respectively.

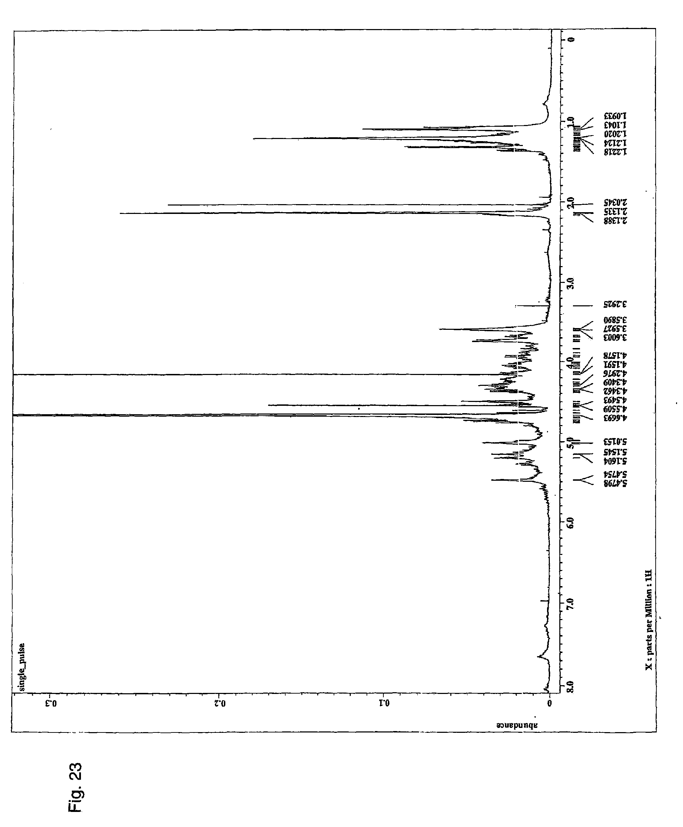

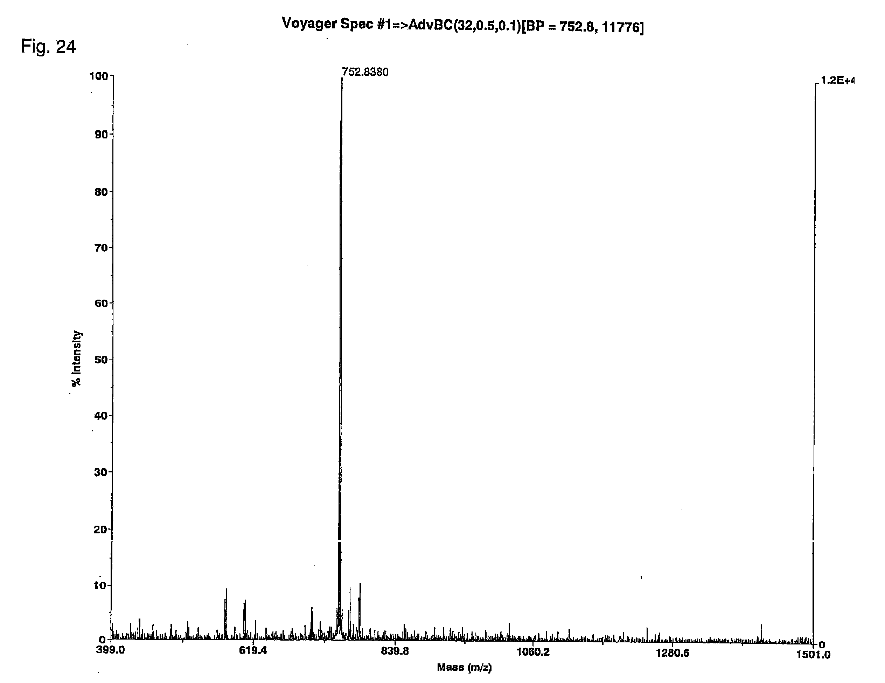

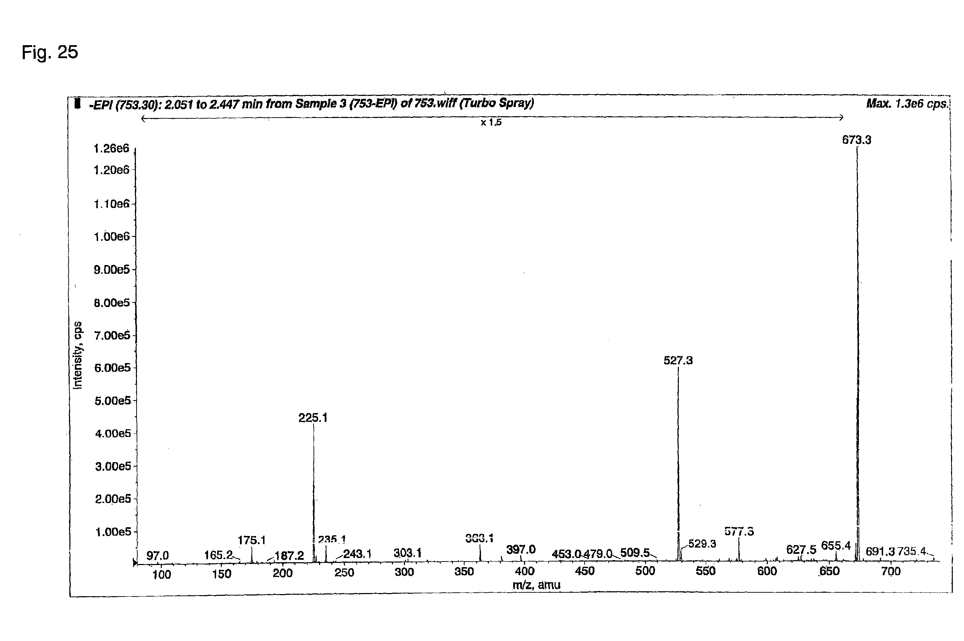

For confirmation, ABEE bound to each oligosaccharide was removed to obtain a pure form of the regenerated oligosaccharide. To be more specific, to 10 mg (100 µl) each of the thus separated, labeled oligosaccharides, 10 µl each of hydrogen peroxide and acetic acid was added and the mixture was left to stand for 24 hours and then solidified to dryness. Among the thus regenerated oligosaccharides, the one obtained from the compound with a molecular weight of 903 was analyzed by ¹H-NMR (Fig. 23), TOF-MS (apparatus: Voyager DE-STR (Applied Biosystems); Ion mode: negative; Mode of operation: reflector; Accelerating voltage: 20 kV; Matrix: 2,5-dihydroxybenzoic acid) (Fig. 24), and ESI-MS/MS (Fig. 25). The results were certainly in agreement with the structural data for formula (VII).

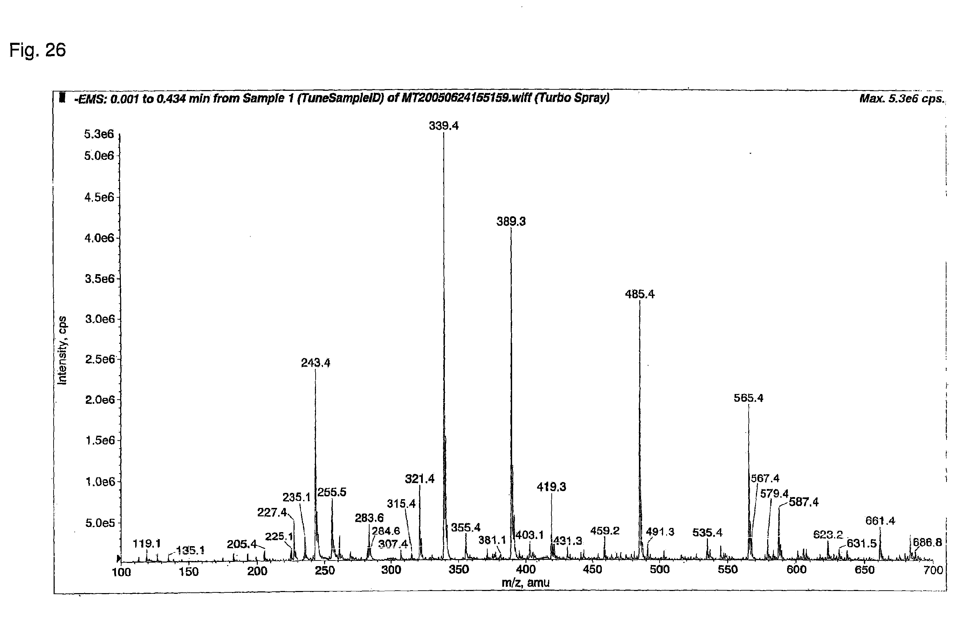

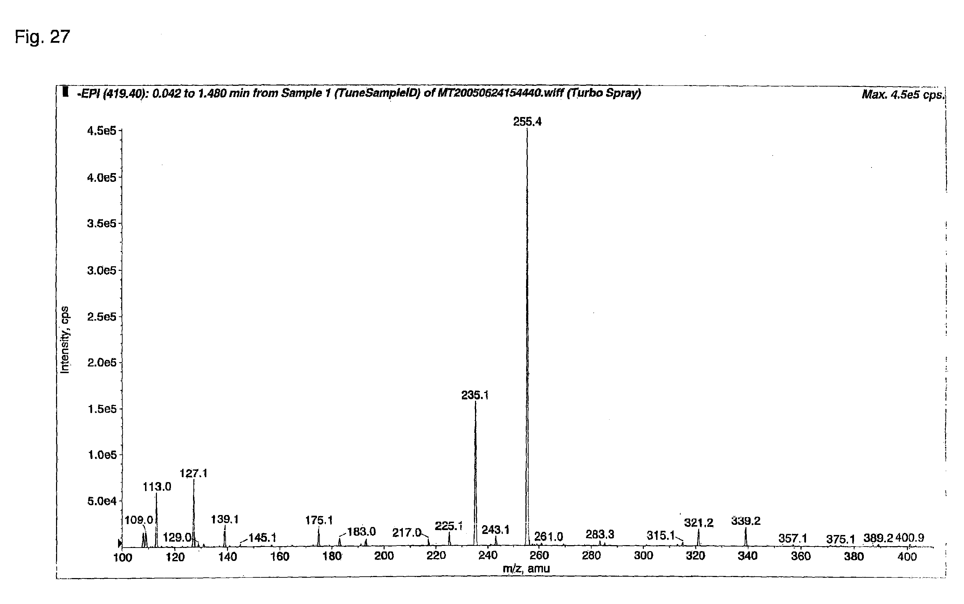

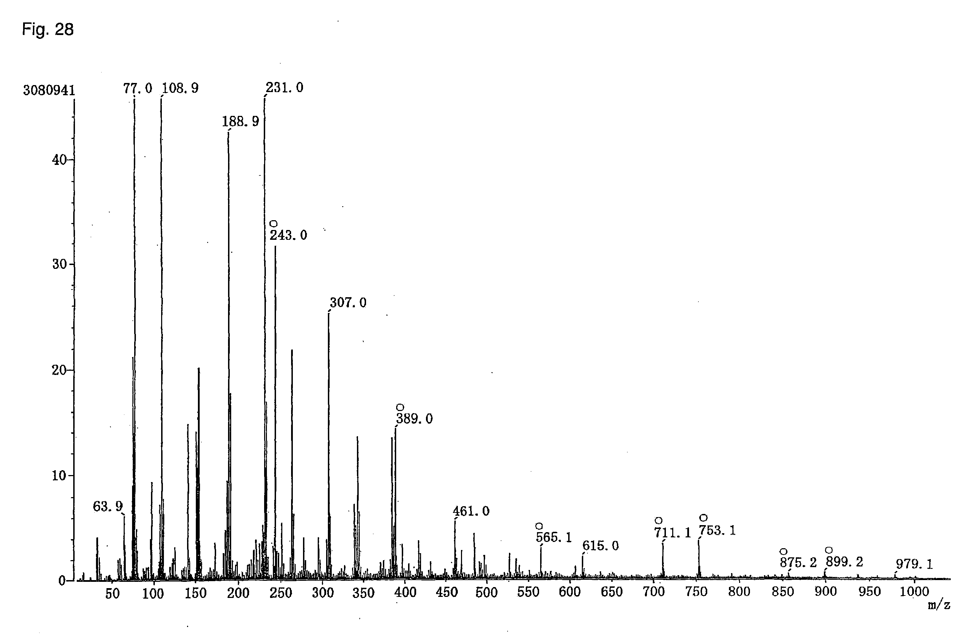

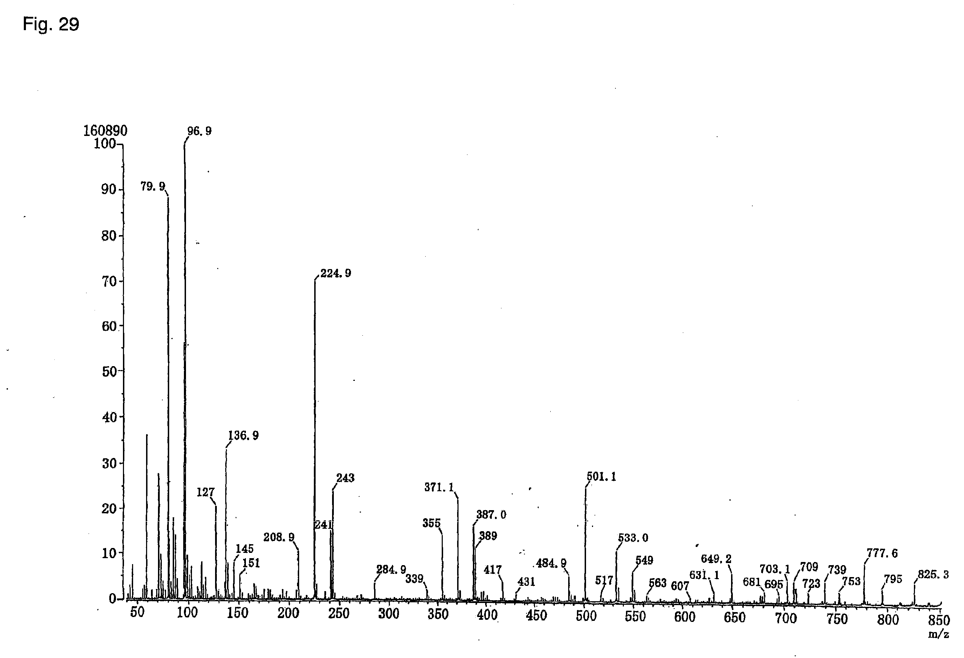

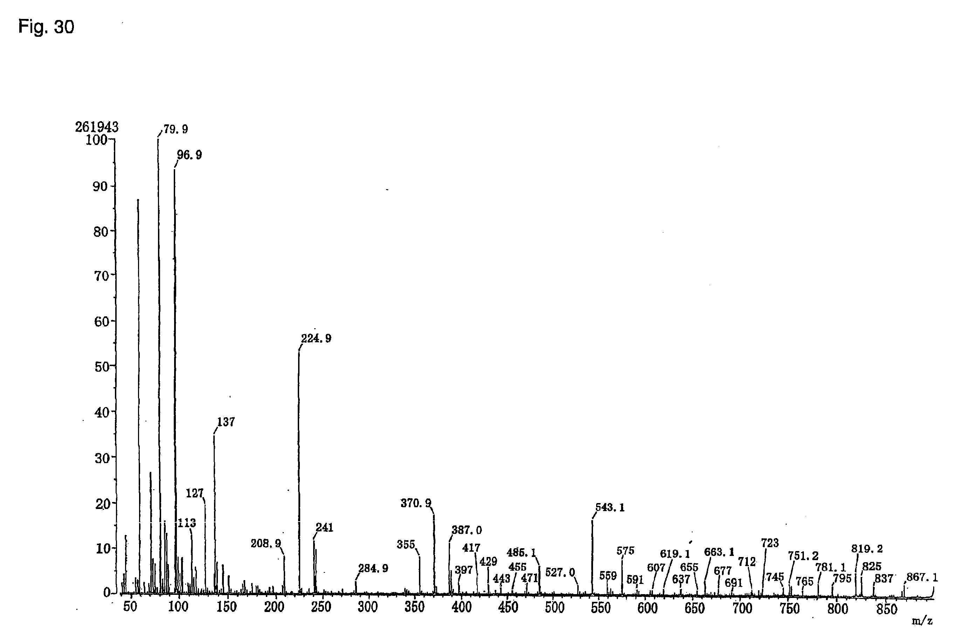

As for the compounds with molecular weights of 420, 858 and 900 (which were respectively compounds (IV), (X) and (XI)) that could not be separated by the method described above, the reaction mixture was analyzed by FAB-MS/MS (apparatus: HX110A/HX110A (JEOL); Ion mode: MS, MS/MS (negative); Xe atom beam: 5 kV; Ion source accelerating potential: 10 kV; Collision energy: 2 keV; Matrix: Glycerol) and ESI-MS-MS to confirm their presence. Fig. 26 shows the FAB-MS chart of (IV); Fig. 27 shows the MS/MS chart of (IV); Fig. 28 shows the FAB-MS charts of (X) and (XI); and Figs. 29 and 30 show the FAB-MS charts of (X) and (XI).

From these results, the following were found: the disaccharide with a molecular weight of 390 was α-L-Fuc-4-O-SO₃⁻-(1→3)-L-Fuc represented by chemical formula (III); the disaccharide with a molecular weight of 420 was α-D-G1cA-(1→2)-L-Fuc represented by chemical formula (IV); the trisaccharide having a molecular weight of 566 was α-L-Fuc-4-O-SO₃⁻-(1→3)-[α-D-GlcA-(1→2)]-L-Fuc represented by chemical formula (V); the tetrasaccharide with a molecular weight of 712 was α-L-Fuc-4-O-SO₃⁻- (1→3) - [α-D-GlcA- (1→2)] -α-L-Fuc-(1→3)-L-Fuc represented by chemical formula (VI); the tetrasaccharide with a molecular weight of 754 was α-L-Fuc-4-O-SO₃⁻- (1→3)-[α-D-GlcA- (1→2)]-α-L-Fuc-4-O-acetyl-(1→3)-L-Fuc represented by chemical formula (VII); the pentasaccharide with a molecular weight of 808 was [α-D-GlcA-(1→2)-α-L-Fuc-(1→3)]-[α-D-GlcA-(1→2)]-α-L-Fuc-(1→3)-L-Fuc represented by formula (VIII); the pentasaccharide with a molecular weight of 850 was [α-D-GlcA-(1→2)-α-L-Fuc-(1→3)]-[α-D-GlcA-(1→2)]-4-O-acetyl-α-L-Fuc-(1→3)-L-Fuc represented by formula (IX); the pentasaccharide with a molecular weight of 858 was α-L-Fuc-4-O-SO₃⁻-(1→3)-α-L-Fuc-(1→3)-[α-D-GlcA-(1→2)]-α-L-Fuc-(1→3)-L-Fuc represented by formula (X); and the pentasaccharide with a molecular weight of 900 was α-L-Fuc-4-O-SO₃⁻-(1→3)-α-L-Fuc-(1→3)-[α-D-GlcA-(1→2)]-α-L-Fuc-4-O-acetyl-(1→3)-L-Fuc represented by formula (XI).

**Table 4: Results of ¹H-NMR and ¹³C-NMR analyses of the fluorescently labeled compound (III)**

| Position | ¹H -NMR (D₂O) δ | ¹³C -NMR (D₂O) MR δ |
|---|---|---|
| ABEE-1 | | 117.3 |
| -2,6 | 7.956 (2H, d, J = 8.9 Hz) | 131.8 |
| -3,5 | 6.997 (2H, d, J = 8.7 Hz) | 115.9 |
| -4 | | 149.2 |
| -C=O | | 168.9 |
| -CH₂ | 4.331 (2H, q, J = 7.2 Hz) | 62.1 |
| -CH₃ | 1.343 (3H, t, *J* = 7.1 Hz) | 13.6 |
| Fuc-1(CH₂) | 3.530 (1H, dd, *J* = 13.4, 4.9 Hz) 3.456 (1H, dd, *J* = 13.5, 8.2 Hz) | 47.8 |
| -2 | 4.113 (1H, dq, *J* 12.0, 3.1 Hz) | 68.7 |
| -3 | 3.774 (1H, dd, *J* = 6.8, 1.5 Hz) | 78.4 |
| -4 | 3.709 (1H, dd, *J* = 6.2, 2.7 Hz) | 73.8 |
| -5 | 4.113 (1H, dq, *J* = 12.0, 3.1 Hz) | 66.1 |
| -CH₃ | 1.194 (3H, d, J = 6.4 Hz) | 18.7 |
| SFuc-1 | 5.081 (1H, d, J = 3.9 Hz) | 99.2 |
| -2 | 3.823 (1H, dd, J = 10.8, 4.1 Hz) | 68.3 |
| -3 | 3.909 (1H, dd, J = 10.5, 3.4 Hz) | 68.6 |
| -4-SO₃- | 4.455 (1H, d, J = 2.7 Hz) | 80.3 |
| -5 | 3.959 (1H, q, J = 6.3 Hz) | 66.8 |
| -CH₃ | 1.081 (3H, d, J = 6.6 Hz) | 15.9 |

**Table 5: Results of ¹H-NMR and ¹³C-NMR analyses of the fluorescently labeled compound (V)**

| Position | ¹H -NMR (D₂O) δ | ¹³C -NMR (D₂O) δ |
|---|---|---|
| ABEE-1 | - | 117.8 |
| -2,6 | 7.794 (2H, d, J = 8.5 Hz) | 131.8 |
| -3,5 | 6.699 (2H, d, J = 8.2 Hz) | 112.4 |
| -4 | - | 152.3 |
| -C=O | - | 169.4 |
| -CH₂ | 4.257 (2H, q, J = 7.1 Hz) | 61.8 |
| -CH₃ | 1.285 (3H, t, J = 7.1 Hz) | 13.7 |
| Fuc-1(CH₂) | 3.595 (1H, dd, J = 14.8, 4.7 Hz) 3.557 (1H, t, J = 3.5 Hz) | 43.6 |
| -2 | 4.076-4.055 (1H, m) | 77.0 |
| -3 | 3.860 (1H, d, J = 8.7 Hz) | 77.3 |
| -4 | 3.729 (1H, d, J = 8.5 Hz) | 73.2 |
| -5 | 4.109 (1H, q, J = 6.5 Hz) | 65.6 |
| -CH₃ | 1.173 (3H, d, J = 6.4 Hz) | 18.6 |
| GlcA-1 | 5.111 (1H, d, J = 3.4 Hz) | 98.5 |
| -2 | 3.556-3.526 (1H, m) | 70.9 |
| -3 | 3.644 (1H, t, J = 9.2 Hz) | 72.6 |
| -4 | 3.489 (1H, t, J = 9.5 Hz) | 71.3 |
| -5 | 4.165 (1H, d, J 10.3 Hz) | 71.2 |
| -COOH | - | 172.8 |
| sFuc-1 | 5.036 (1H, d, *J* =3.7 Hz) | 100.6 |
| -2 | 3.768 (1H, dd, J = 10.8, 3.7 Hz) | 68.2 |
| -3 | 3.843 (1H, t, J = 5.5 Hz) | 68.6 |
| -4-SO₃- | 4.371 (1H, m) | 80.3 |
| -5 | 3.911 (1H, q, J = 6.4 Hz) | 66.9 |
| -CH₃ | 1.038 (3H, d, J = 6.4 Hz) | 15.8 |

**Table 6: Results of ¹H-NMR and ¹³C-NMR analyses of the fluorescently labeled compound (VI)**

| Position | ¹H -NMR (D₂O) δ | ¹³C -NMR (D₂O) δ |
|---|---|---|
| ABEE-1 | - | 123.8 |
| -2,6 | 7.952 (2H, dd, J = 6.9, 2.1 Hz) | 131.4 |
| -3,5 | 7.087 (2H, dd, J = 6.9, 1.8 Hz) | 117.3 |
| -4 | - | 146.6 |
| -C=O | - | 168.5 |
| -CH₂ | 4.286 (2H, q, J = 7.0 Hz) | 62.7 |
| -CH₃ | 1.292 (3H, t, J = 7.1 Hz) | 13.6 |
| Fuc-1(CH₂) | 3.526 (1H, dd, J = 13.4, 8.6 Hz) 3.572-3.556 (1H, m) | 48.9 |
| -2 | 4.009-3.985 (1H, m) | 68.6 |
| -3 | 3.783 (1H, d, J = 2.7 Hz) | 76.8 |
| -4 | 3.708 (1H, dd, J = 6.0, 3.7 Hz) | 74.4 |
| -5 | 4.009-3.985 (1H, m) | 66.6 |
| -CH₃ | 1.185 (3H, d, J = 6.4 Hz) | 18.9 |
| Fuc-1 | 5.049 (1H, d, J = 3.7 Hz) | 97.7 |
| -2 | 4.117 (1H, d, J = 9.8 Hz) | 70.9 |
| -3 | 4.033 (1H, dd, J = 10.6, 2.9 Hz) | 72.5 |
| -4 | 3.792 (1H, d, J = 2.7 Hz) | 66.8 |
| -5 | 3.430 (1H, q, J = 6.7 Hz) | 67.2 |
| -CH₃ | 0.900 (3H, d, J = 6.6 Hz) | 15.3 |
| SFuc-1 | 5.069 (1H, d, J = 3.9 Hz) | 93.2 |
| -2 | 3.821 (1H, dd, J = 10.4, 3.8 Hz) | 68.1 |
| -3 | 3.970 (1H, dd, J = 10.4, 3.1 Hz) | 68.9 |
| -4-SO₃- | 4.512 (1H, d, J = 3.0 Hz) | 80.8 |
| -5 | 4.455 (1H, q, J = 6.6 Hz) | 66.6 |
| -CH₃ | 1.232 (3H, d, J = 6.4 Hz) | 16.2 |
| GlcA-1 | 5.266 (1H, d, J = 3.7 Hz) | 99.6 |
| -2 | 3.602 (1H, dd, J = 9.7, 4.0 Hz) | 70.6 |
| -3 | 3.643 (1H, t, J = 9.4 Hz) | 72.7 |
| -4 | 3.587-3.556 (1H, m). | 71.2 |
| -5 | 4.108 (1H, t, J = 5.4 Hz) | 71.6 |
| -COOH | - | 172.92 |

**Table 7: Results of ¹H-NMR and ¹³C-NMR analyses of the fluorescently labeled compound (VII)**

| Position | ¹H -NMR (D₂O) δ | ¹³C -NMR (D₂O) δ |
|---|---|---|
| ABEE-1 | - | 122.4 |
| -2,6 | 7.953 (2H, dd, J = 7.0, 1.7 Hz) | 131.9 |
| -3,5 | 7.021 (2H, d, J = 8.9 Hz) | 116.4 |
| -4 | - | 148.2 |
| -C=O | - | 168.5 |
| -CH₂ | 4.290 (2H, ddd, J = 14.3, 7.1, 1.3 Hz) | 62.2 |
| -CH₃ | 1.299 (3H, t, J = 7.1 Hz) | 13.6 |
| Fuc-1 (CH₂) | 3.604-3.573 (1H, m) 3.488 (1H, dd, J = 13.5, 8.0 Hz) | 48.0 |
| -2 | 3.977-3.953 (1H, m) | 68.9 |
| -3 | 3.766 (1H, q, J = 2.9 Hz) | 76.6 |
| -4 | 3.713 (1H, td, J = 5.8, 3.8 Hz) | 74.3 |
| -5 | 3.977-3.953 (1H, m) | 66.7 |
| -CH₃ | 1.191 (3H, d, J = 6.4 Hz) | 18.8 |
| FucAc-1 | 5.078 (1H, d, J = 2.7 Hz) | 97.5 |
| -2 | 4.120 (1H, d, J = 1.8 Hz) | 71.3 |
| -3 | 4.120 (1H, d, J = 1.8 Hz) | 69.9 |
| -4 | 4.971 (1H, s) | 68.3 |
| -5 | 3.319 (1H, d, J = 6.6 Hz) | 65.8 |
| -COCH₃ | 2.058 (3H, s) | 173.5/20.1 |
| -CH₃ | 0.764 (3H, d, J = 6.4 Hz) | 15.1 |
| GlcA-1 | 5.231 (1H, d, J = 3.7 Hz) | 99.7 |
| -2 | 3.604-3.573 (1H, m) | 70.5 |
| -3 | 3.644 (1H, t, J = 9.4 Hz) | 72.8 |
| -4 | 3.556 (1H, d, J = 8.9 Hz) | 71.2 |
| -5 | 4.102 (1H, t, J = 4.9 Hz) | 71.5 |
| -COOH | | 173.0 |
| SFuc-1 | 4.947 (1H, d, J = 4.1 Hz) | 93.1 |
| -2 | 3.740 (1H, d, J = 3.9 Hz) | 67.8 |
| -3 | 3.858 (1H, dd, J = 10.5, 3.2 Hz) | 68.9 |
| -4-SO₃- | 4.501 (1H, d, J = 3.9 Hz) | 80.8 |
| -5 | 4.485 (1H, t, J = 7.4 Hz) | 66.2 |
| -CH₃ | 1.267 (3H, d, J = 6.6 Hz) | 16.2 |

### EXAMPLE 5

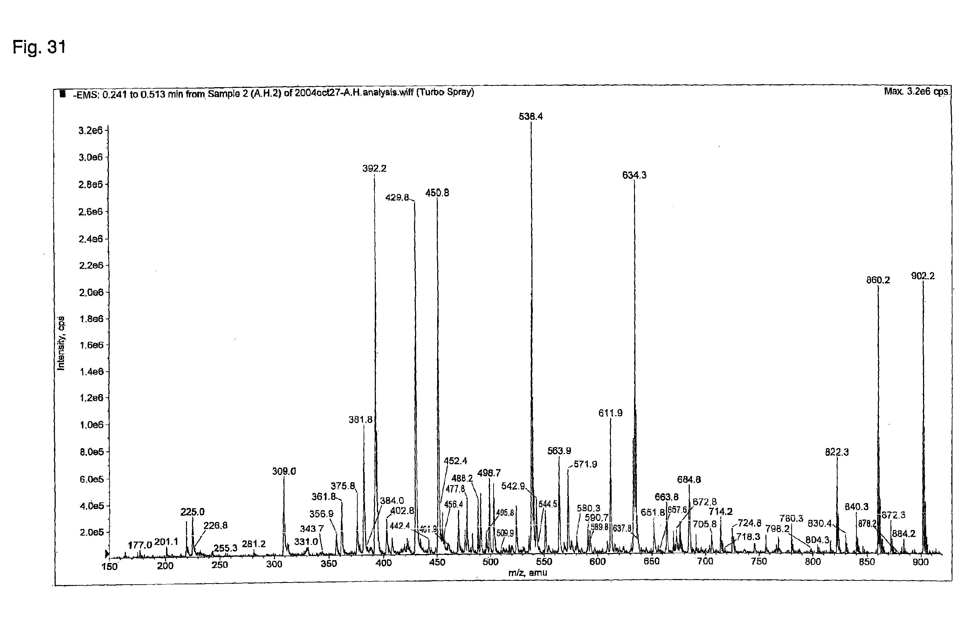

To 100 g of an algal body of Okinawa Nemacystus decipiens, 1000 ml of 2 N HCl was added and the mixture was subjected to acid hydrolysis at 50-100°C for one hour. The resulting extracts were cooled, filtered by suction, electrodialyzed (desalted) and freeze-dried to give 2 g of fucoidan fractions. The obtained fucoidan fractions were fluorescently labeled with ABEE and analyzed by ESI-MS (4000Q TRAP LC/MS/MS system (Applied Biosystems); analysis conditions = Polarity: Negative ion mode; Declustering potential: -50 V; Collision energy: -10 eV; Temperature: 550°C) to give the results shown by chart in Fig. 31; they verified the presence of fucoidan oligosaccharides represented by formulae (I) to (XI).

### EXAMPLE 6

### Synergism of the Combination of Fucoidan Oligosaccharide Compounds with Lactic Acid Bacteria

### IFN-γ inducible activity on mouse splenocytes

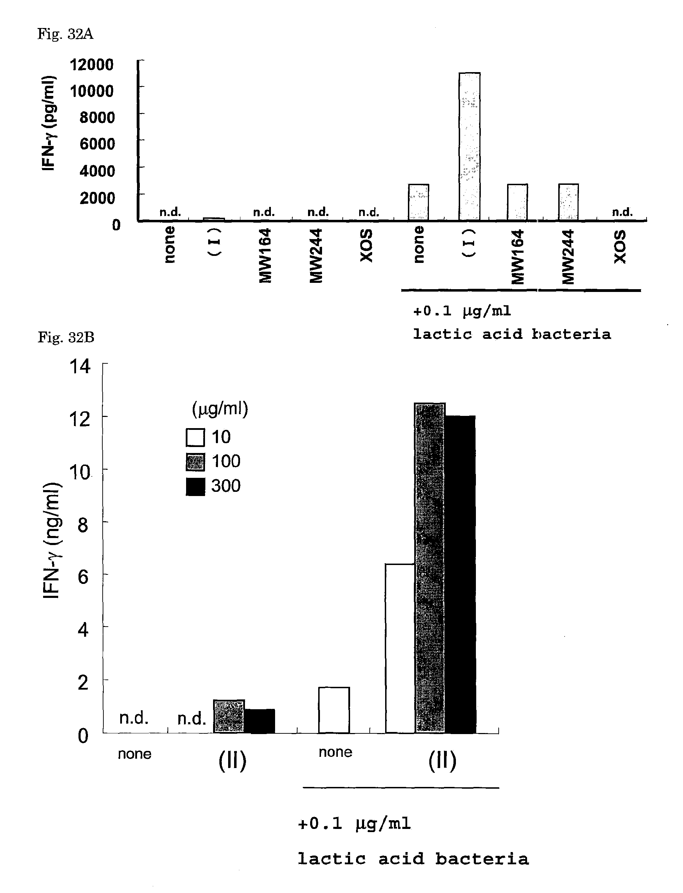

Splenocytes were prepared as in Example 3 and 5 x 10⁶ of them per ml were placed in wells, into each of which a fucoidan oligosaccharide (compound (I)) having a molecular weight of 340 was also put at a concentration of 100 µg/ml.
As controls, fucose and sulfated fucose (with respective MWs of 164 and 244), as well as xylooligosaccharides (XOS) were used in equal amounts. Thirty minutes later, dead cells of a lactic acid bacteria (Lactobacillus pentosus: FERM ABP-10028) were put into each well in an amount of 0.1 µg/ml. After 24-hr culture, the supernatant of the culture was recovered and the production of IFN-γ was quantified with an ELISA kit (OptEIA, BD Pharmingen). The results are shown in Fig. 32A. In order to verify the effect of the saccharide without lactic acid bacteria, the same operation was performed without using the lactic acid bacteria.

Further, the same experiment was conducted for compound (II), as was done for compound (I). The doses of compound (II) used were 10, 100 and 300 µg/ml in wells.

As for the compound (I), it conferred a very strong IFN-γ inducing ability when it was used in combination with the lactic acid bacteria (Fig. 32A). This strong action was unpredictable from the results of using the compound (I) and the lactic acid bacteria individually (see the second bar from the left and the fifth bar from the right in Fig. 32A), thus supporting the synergism of using the compound (I) and the lactic acid bacteria in combination. The same synergism was recognized for compound (II) and the lactic acid bacteria (Fig. 32B). No such activity was found in the constituents fucose and sulfated fucose, and the widely used prebiotics xylooligosaccharides were also inactive. It therefore became clear that the fucoidan oligosaccharides of the present invention synergistically activate splenocytes when used in combination with lactic acid bacteria and hence are very useful in modulating the immune function of the living body.

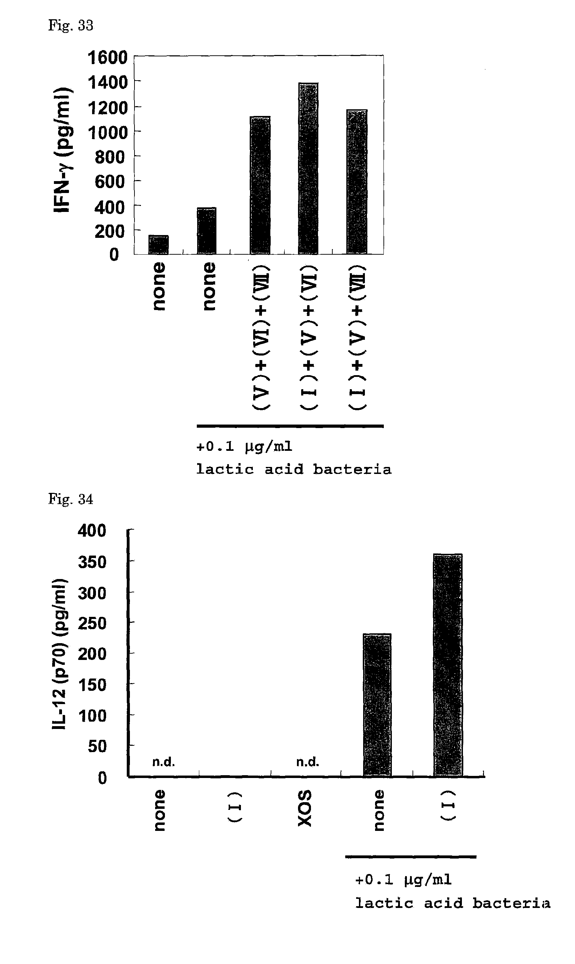

Other samples of splenocytes were similarly prepared and three compounds randomly selected from among the compounds (I) to (VII) and mixed together in equal amounts were added at a concentration of 50 µg/ml. Thirty minutes later, dead cells of lactic acid bacteria (Lactobacillus pentosus: FERM ABP-10028) were put into each well in an amount of 0.1 µg/ml. After 24-hr culture, the supernatant of the culture was recovered and the production of IFN-γ was quantified. The results are shown in Fig. 33; high IFN-γ production was observed when there were used a mixture of (V), (VI) and (VII), a mixture of (I), (V) and (VI), and a mixture of (I), (V) and (VII). It was therefore clear that even when some of the compounds (I) - (XI) were used in admixture, an elevated immunomodulatory activity was achieved by using them in combination with lactic acid bacteria.

### EXAMPLE 7

### The IL-10 and IL-12 Inducible Activity and the CTL Activity Potentiating Action of Fucoidan Oligosaccharides on Mouse Derived Dendritic Cells

Bone marrow cells were isolated from the femoral bones of BALB/c mice (8-wk old, male; Japan SLC, Inc.). In an RPMI 1640 medium supplemented with 10% FBS, 20 ng/ml of GM-CSF (Peprotec), and 20 ng/ml of IL-3 (Peprotec), the isolated bone marrow cells were suspended at a density of 1 x 10⁶ cells/ml and cultured on a 24-well plate. At days 3 and 5 of the culture, the medium was changed to give adherent immature dendritic cells. The recovered dendritic cells were preliminarily treated with compound (I), (II), (III), (V), (VI) or (VII) at a concentration of 50 µg/ml. Thirty minutes after the preliminary treatment, dead cells of lactic acid bacteria (Lactobacillus pentosus: FERM ABP-10028) were put into each well in an amount of 0.1 µg/ml. After 2-day culture, the supernatant of the culture and the cells were recovered. In order to verify the effect of the saccharide compounds used alone, the same operation as above was performed without adding the lactic acid bacteria.

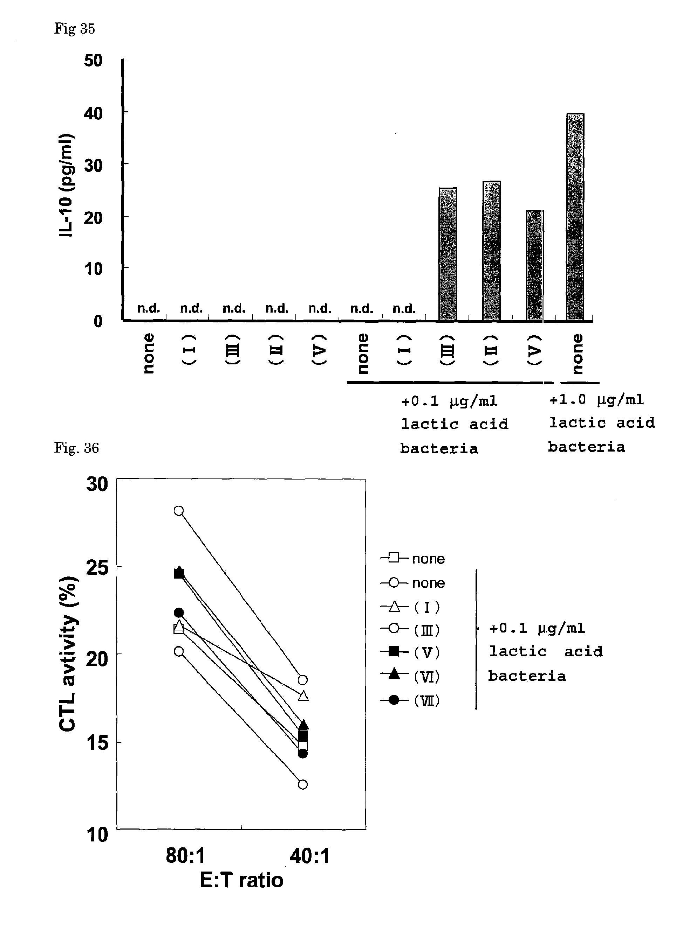

The recovered supernatant of the culture was assayed for IL-12 and IL-10 using an ELISA kit. The results are shown in Figs. 34 and 35. Compound (I) induced IL-12 synergistically when it was used in combination with the lactic acid bacteria (Fig. 34). Compounds (II), (III) and (V) were also found to induce IL-10 when they were used in combination with the lactic acid bacteria (Fig. 35). From these results, it became clear that the fucoidan oligosaccharides discovered in the present invention are capable of modulating the immune function of a living body in both a positive and a negative direction (either activating lowered immunity or suppressing an excessive immune reaction). No such action was recognized in the xylooligosaccharides.

The recovered cells were counted and their density was adjusted to 1 x 10⁵ cells/ml; then, 1 ml of the cell suspension was treated with mitomycin C (50 µg/ml) to effect reaction at 37°C for 30 minutes. After the reaction, mitomycin C was removed by washing with PBS and C57BL/6 mouse splenocytes (5 x 10⁶ cells/ml) that were prepared in the usual manner were added in an amount of 1 ml and mixed culture was performed for 4 days. After the culture, the cells were recovered and those P-815 cells which have alloantigens of the C57BL/6 mice (5000 cells/100 µl) were selected as targets for reaction which was performed for 4 hours at E:T ratios of 40 : 1 and 80:1. The number of killed P-815 cells was counted by flow cytometry to determine the CTL activity (Fig. 36). It was found that compounds (I), (III), (V), (VI) and (VII) could also potentiate the CTL activity when used in combination with lactic acid bacteria.

The results of Example 7 also show that the fucoidan hydrolysates of the present invention, if combined with lactic acid bacteria, provide a synergistically enhanced immunomodulatory or immuno-stimulating action.

### EXAMPLE 8

### Synergistically Enhanced IL-12 Production from Fucoidan Hydrolysates Used in Combination with Lactic Acid Bacteria Having Little Immunomodulatory Activity

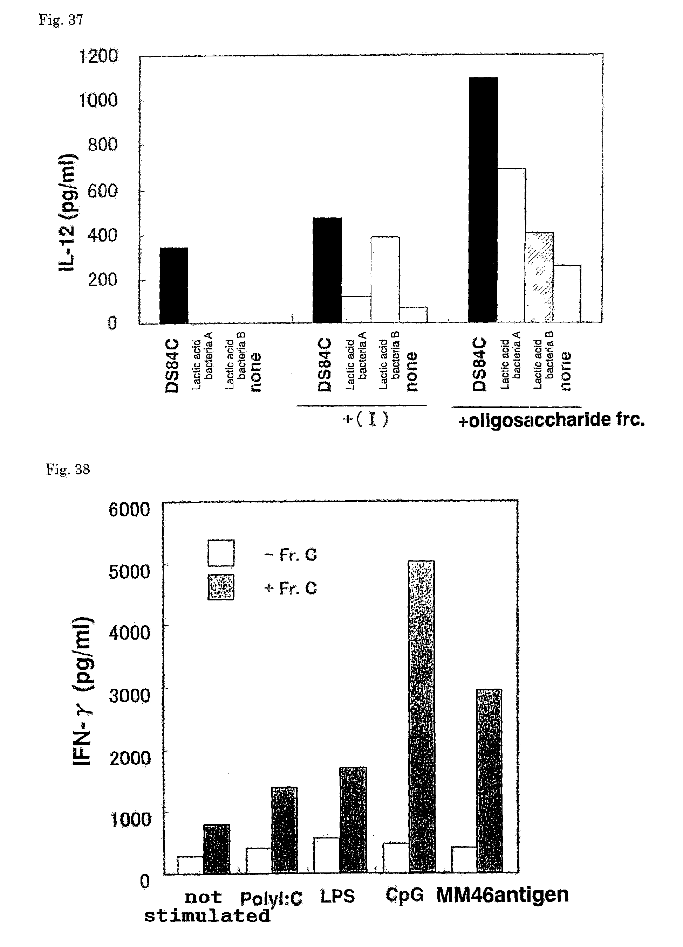

BALB/c mice (8-wk old, male; Japan SLC, Inc.) were administered intraperitoneally with a 4.05% thioglycolate medium (Difco) and 4 days later, the intraperitoneally invasive cells were recovered using 10 ml of PBS. Thereafter, the cell density was adjusted to 1 x 10⁶ cells/ml and culture was effected on a 24-well plate. Three hours after the start of culture, the medium was changed to remove the suspended cells and produce macrophages. Into each well, the compound (I) and the oligosaccharide fraction described in Example 3 were added at a concentration of 100 µg/ml; 30 minutes later, lactic acid bacteria having strong immunomodulatory activity (Lactobacillus pentosus, strain DS 84C; FERM ABP-10028 accepted by the International Patent Organism Depositary, the National Institute of Advanced Industrial Science and Technology on May 26, 2004) or either one of two lactic acid bacteria having little immunomodulatory activity (commercial Lactobacillus pentosus (hereinafter referred to as lactic acid bacteria A) and commercial Lactobacillus plantarum (hereinafter referred to as lactic acid bacteria B)) was added at a concentration of 0.1 µg/ml and 24-hr culture was performed. Thereafter, the supernatant of the culture was recovered and the production of IL-12 was quantified (see Fig. 37). For comparison, the same operation was performed using lactic acid bacteria alone (each of the three strains of lactic acid bacteria was used). Neither lactic acid bacteria A nor B showed a detectable level of IL-12 inducing activity on their own; however, when those strains were used in combination with the compound (I) or the oligosaccharide fraction, a significantly increased IL-12 inducing activity was obtained. Therefore, it became clear that the fucoidan hydrolysates obtained in the present invention could exhibit a strong immunomodulatory activity in a living body even when they were used in combination with lactic acid bacteria having no immunomodulatory activity.

### EXAMPLE 9

### Synergism with Substances Other Than Lactic Acid Bacteria

Splenocytes were isolated from C57BL/6 mice (8-wk old, male, CHARLES RIVER LABORATORIES JAPAN, INC.) and their density was adjusted to 5 x 10⁶ cells/ml for culturing on a 24-well plate. The Fr. C fraction of Example 3 was added into each well in an amount of 30 µg/ml. Thirty minutes: later, the following substances having the immuno-stimulating or immunomodulatory activity were individually added into the wells at the indicated concentrations: 5 µg/ml of PolyI:C (SIGMA); 2 ng/ml of LPS (SIGMA); 3 µg/ml of CpG-ODN (5'-TCCATGACGTTCCTGATGCT-3': obtained from Integrated DNA Technologies, Inc.); 50 ng protein/ml of MM46-tumor antigen (protein extracted from the homogenate of MM46 mammary cancer cells in HANK's solution (nacalai tesque): provided from Teikyo University Institute of Medical Mycology); and 0.25 µg/ml of ConA (Wako Pure Chemical Industries, Ltd.). After 24-hr culture, the supernatant of the culture was recovered and the production of IFN-γ was quantified with an ELISA kit (OptEIA, BD Pharmingen). The results are shown in Fig. 38. For comparison, the same operation was performed without using Fr. C. The immunomodulatory substances could hardly induce IFN-γ at the concentrations used in the experiment. However, when they were used in combination with Fr. C which is a fucoidan hydrolysate (oligosaccharides), strong IFN-γ inducing ability was obtained. It therefore became clear that the fucoidan hydrolysates as well as the oligosaccharides obtained therefrom had their immuno-stimulating or immunomodulatory activity enhanced synergistically not only when they were used in combination with lactic acid bacteria but also with other immuno-stimulating materials.

### EXAMPLE 10

### NK Activity Potentiating Effect (In vivo)

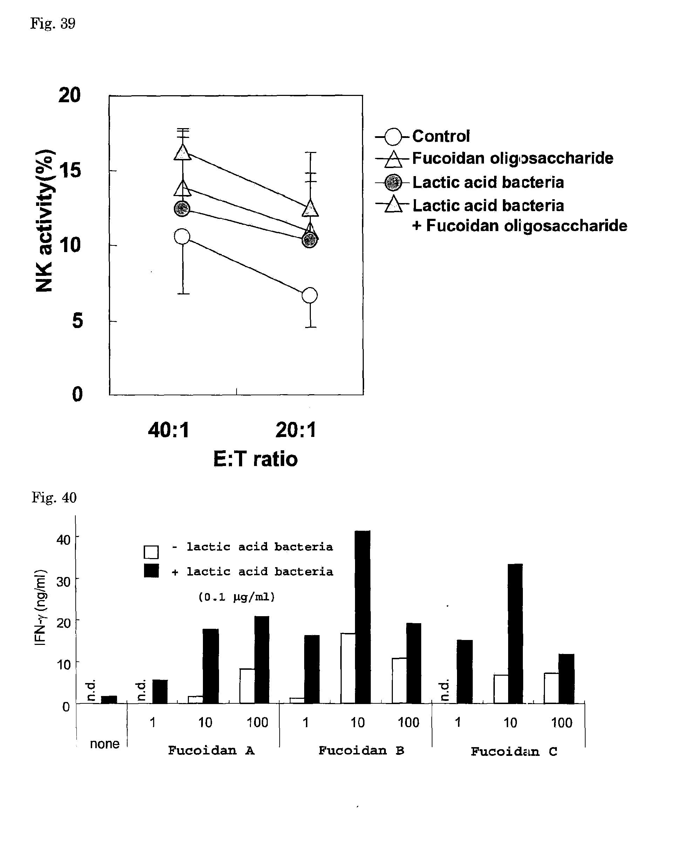

C57BL/6 mice (8-wk old, male, CHARLES RIVER LABORATORIES JAPAN, INC.) were divided into four groups, three of which were let to take the following meals ad libitum as they were mixed in drinking water: fucoidan hydrolysate alone (the sulfated saccharide fraction, 500 mg/kg, described in Example 3: Fig. 39, "fucoidan oligosaccharide"); lactic acid bacteria alone (0.2 mg/kg); and fucoidan hydrolysate + lactic acid bacteria (Fig. 39, "lactic acid bacteria + fucoidan oligosaccharide"). The other group was a control group and allowed to drink tap water. One week after the mice started eating or drinking, splenocytes were isolated from them by a standard procedure and their NK activity was measured with Yac-1 cells (5000 cells/100 µl) being selected as targets. Reaction was performed for 4 hours at E:T ratios of 20:1 and 40:1 and the number of killed Yac-1 cells was counted by flow cytometry to calculate the NK activity (Fig. 39). The NK activity was potentiated in the animals that ingested the fucoidan oligosaccharide fraction obtained from fucoidan. A further increase in NK activity was achieved by using the oligosaccharide fraction in combination with the lactic acid bacteria. Thus, it was also verified in vivo that the immune function can be activated by ingesting the combination of the fucoidan hydrolysate and lactic acid bacteria.

### EXAMPLE 11

### Synergism of the Combination of Fucoidan with Lactic Acid Bacteria

### IFN-γ inducible activity on mouse splenocytes

Splenocytes were prepared as in Example 3 and 5 × 10⁶ of them per ml were placed in wells, into each of which a fucoidan sample A (South Product Ltd.), a fucoidan sample B (Okinawa fermentation technology CO., Ltd.), or a fucoidan sample C (which was prepared in Example 1) was also put at a concentration of 1, 10, or 100 µg/ml. Thirty minutes after the addition of the fucoidan samples, dead cells of lactic acid bacteria (Lactobacillus pentosus: FERM ABP-10028) were put into each well in an amount of 0.1 µg/ml. After 24-hr culture, the supernatant of the culture was recovered and the production of IFN-γ was quantified with an ELISA kit (OptEIA, BD Pharmingen). The results are shown in Fig. 40. In order to verify the effect of the fucoidan samples used alone, the same operation was performed without using the lactic acid bacteria.

When each of the three fucoidan samples was used alone, it conferred an IFN-γ inducing ability. Further, the ability was enhanced beyond expectations when it was used in combination with the lactic acid bacteria. It therefore became clear that fucoidan synergistically activates splenocytes when used in combination with lactic acid bacteria and hence is very useful in modulating the immune function of a living body.

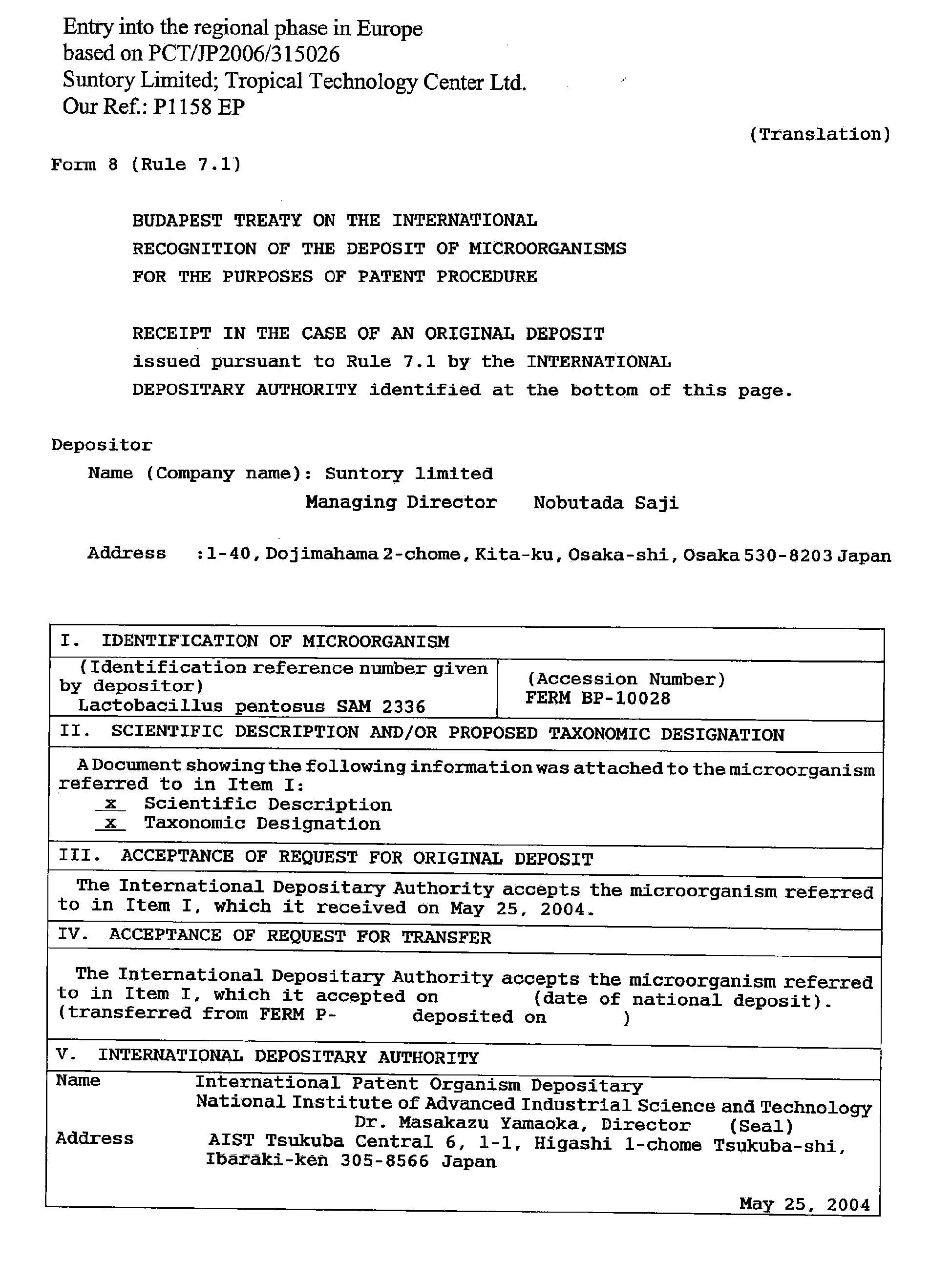

## Claims

1. A composition comprising fucoidan or a fucoidan hydrolysate and an immuno-stimulating material.

2. The composition as recited in claim 1, wherein the hydrolysate is obtained by hydrolyzing fucoidan with 0.1 - 6.0 N acid at 25 - 100 °C for 0.25 - 2.5 hours.

3. The composition as recited in claim 2, wherein the hydrolysate is obtained by hydrolyzing fucoidan with 0.5 - 4.0 N acid at 30 - 90 °C for 0.5 - 2.0 hours.

4. The composition as recited in any one of claims 1 - 3, wherein the hydrolysis is effected with hydrochloric acid or sulfuric acid.

5. The composition as recited in any one of claims 1 - 4, wherein the hydrolysate is an oligosaccharide.

6. The composition as recited in claim 5, wherein the oligosaccharide is a di-, tri-, tetra- or penta-saccharide that are composed of at least one member of the group consisting of fucose, sulfated fucose, acetylated fucose, and glucuronic acid.

7. The composition as recited in claim 6, which contains as the oligosaccharide at least one member of the group consisting of the compounds represented by the following structural formulae (I), (II), (III), (IV), (V), (VI), (VII), (VIII), (IX), (X), and (XI): 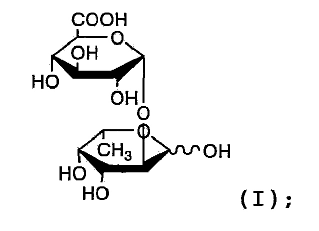 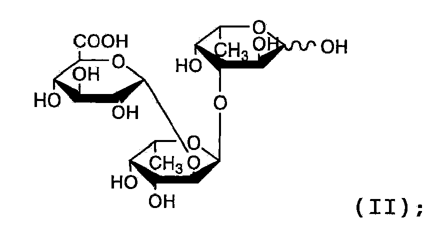 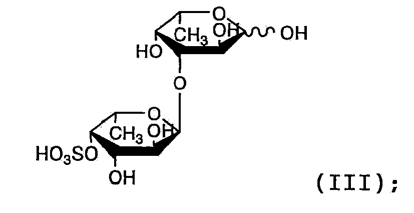 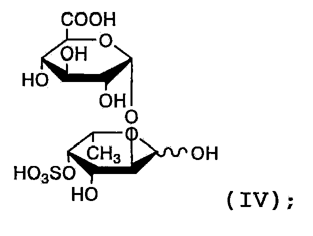 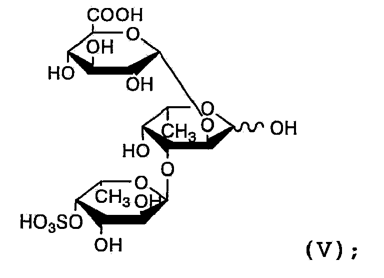 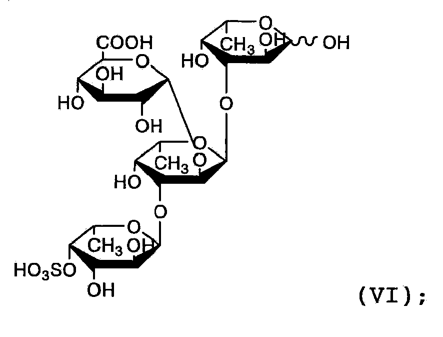 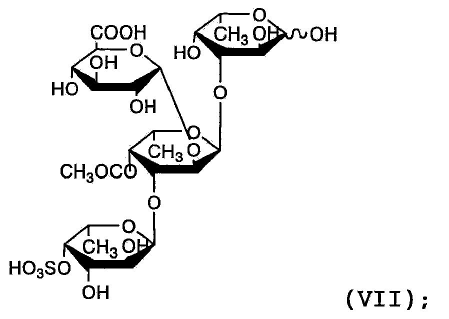 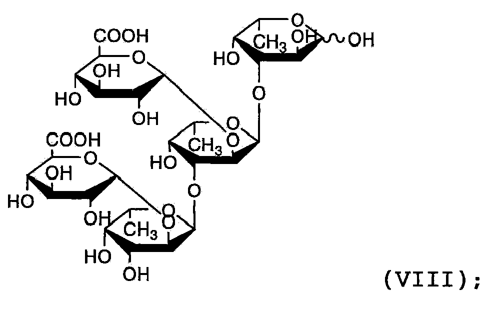 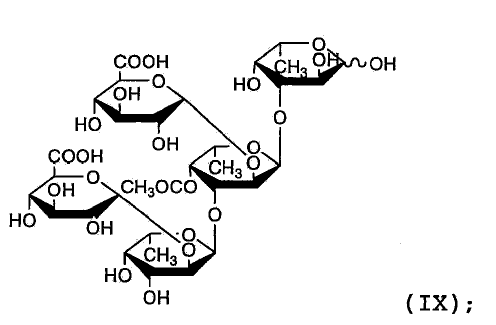 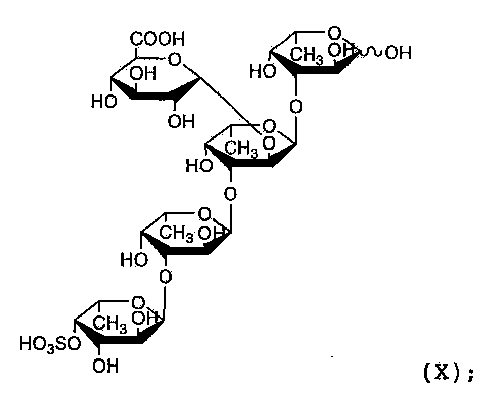 and

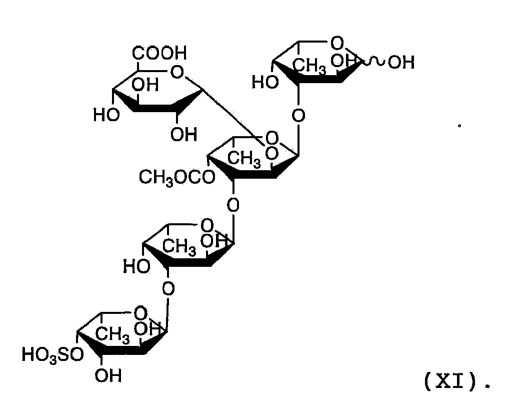

8. The composition as recited in any one of claims 1 - 7, wherein the immuno-stimulating material is at least one member of the group consisting of microorganisms such as lactic acid bacteria, yeasts and fungi; mushrooms such as *shiitake* mushroom, hen-of-the-woods, Ganoderma lucidum, and Agaricus blazei; nucleic acid derived substances such as CpG motif containing immunogenic oligodeoxynucleotide (CpG-ODN) and PolyI:C; lipopolysaccharides (LPS) and β-glucan; sea weeds; Chinese herbal medications; cancer vaccines such as MM46-tumor antigen; concanavalin A (ConA); and BRMs (biological response modifiers).

9. The composition as recited in claim 8, wherein the immuno-stimulating agent is a lactic acid bacterium.

10. An immunomodulatory agent comprising the composition as recited in any one of claims 1 - 9.

11. A food or a drink incorporating the composition as recited in any one of claims 1 - 9, added thereto.

12. A pharmaceutical composition comprising the composition as recited in any one of claims 1 - 9.

13. A cosmetic comprising the composition as recited in any one of claims 1 - 9.


SEQUENCE LISTING

<110> Suntory limited, Tropical Technology Center limited

<120> Compositions comprising fucoidan or a fucoidan hydrolysate and an immuno-stimulating material

<130> YCT-1176

<150> JP 2005-222254
<151> 2005-07-29

<160> 1

<170> PatentIn version 3.1

<210> 1
<211> 20
<212> DNA
<213> Artificial Sequence

<220>
<223> CpG-ODN

<400> 1
tccatgacgt tcctgatgct 20